# EUROPEAN PATENT APPLICATION

(11) **EP 3 939 665 A1**
(43) Date of publication of application: **19.01.2022**
(21) Application number: 20305817.7
(22) Date of filing: 15.07.2020
(51) Int. Cl.: A61P 43/00, C12N 9/10, C12P 19/44, C12N 15/70

(54) **ISOLATED GENE CODING FOR THE ENZYME GLYCOSYL TRANSFERASE 2 FROM PYROCOCCUS HORIKOSHII OT3 OR ITS HOMOLOGS FROM HYPERTHERMOPHILIC ARCHAEA, HOST CELL EXPRESSING IT AND ITS USE IN A PROCESS FOR PRODUCING SULFATED GLYCOSAMINOGLYCANS**

(71) Applicant: Université de Rennes 1, 35000 Rennes (FR); École Nationale Supérieure De Chimie, 35000 Rennes (FR); Institut National des Sciences Appliquées de Rennes, 35700 Rennes (FR); Centre National de la Recherche Scientifique, 75016 Paris (FR); Lebanese University, 99999 Beyrouth (LB)
(72) Inventor: TRANCHIMAND, Sylvain, 35220 Châteaubourg (FR); BENVEGNU, Thierry, 35000 Rennes (FR); CHAMIEH, Hala, 99999 Beyrouth (LB); AMIN, Khadija, 99999 Tripoli (LB); TAHA, Samir, 99999 Beyrouth (LB); ABDEL-RAZZAK, Ziad, 99999 Beyrouth (LB)
(74) Representative: Novagraaf Technologies

(57) **Abstract**

The present invention relates to an isolated gene coding for the enzyme Glycosyl transferase 2 from *Pyrococcus horikoshii* OT3, the nucleotide sequence of said gene comprising or consisting of sequence SEQ ID NO : 1, or its homologs from hyperthermophilic *Archaea.*

The present invention also relates to an isolated enzyme Glycosyl transferases 2 encoded by the isolated gene, and to a plasmid vector comprising a gene coding for the enzyme Glycosyl transferase 2 from *Pyrococcus horikoshii* OT3, the nucleotide sequence of said gene comprising or consisting of the sequence SEQ ID NO : 1, or its homologs from hyperthermophilic *Archaea.*

The invention also relates to a host cell comprising a recombinant polynucleotide encoding Glycosyl transferase 2 from *Pyrococcus horikoshii* OT3, or its homologs from hyperthermophilic Archaea, to a process for producing sulfated glycosaminoglycans (GAGs), and to sulfated glycosaminoglycans obtained by the process and their use for producing pharmaceutical, cosmetic, nutraceutical or food products.

SEQUENCE LISTING

<110>  UNIVERSITÉ DE RENNES 1
       \220ÉCOLE NATIONALE SUPÉRIEURE DE CHIMIE
       \220INSTITUT NATIONAL DES SCIENCES APPLIQUÉES DE RENNES
       CENTRE NATIONAL DE LA RECHERCHE SCIENTIFIQUE
       LEBANESE UNIVERSITY

<120>  Isolated gene coding for the enzyme Glycosyl transferase 2 from
       Pyrococcus horikoshii OT3 or its homologs from hyperthermophilic
       Archaea, host cell expressing it and its use in a process for
       producing sulfated glycosaminoglycans

<130>  BNT229493EP00

<160>  27

<170>  PatentIn version 3.5

<210>  1
<211>  945
<212>  DNA
<213>  Pyrococcus horikoshii

<400>  1
gtgagcataa taatcccagc ttataatgaa gaggagaaca taaaaagagc aattaaagct      60

gctctttctc aggattatcc agttagcgag gtaatagttg tggacgatgg gagcgaggat     120

ggaacgtacg agagggccaa ggaaattaaa gatcatagac ttaaggtact gaagatagat     180

cataggggaa aagcgggggc gataaatgag ggtcttaaaa tcgccaaggg aagtgtgatc     240

gtaactaccg atgcggactc tttcatgagc aaggatgcag ttaggaggtt agttgagagg     300

ttttactccg atgatgttgt tgcagttggg ggccaaataa gggtcatcgt tgaatccttc     360

atgacgctcg ttcaggatat agagcacctg agaattgcga tgtataggag agggaaggag     420

cttgaaaatt taagcttagc ccccggtccc ctttccgctt tcagaaggga tgttctcgaa     480

aagatcggtg ggatacagcg tagtatagtt gaagattacg ccacgactaa gattgtaaag     540

aagtatggaa gggttgttta cgcgccgaag gctaaactct acactagaat gcctatcacc     600

cttcgagatc tatggaggca acgaaagaga tggttcttgg gggatcttca gcaccataac     660

ctgaatgacc ttctgaatct actccttggg gatgtaatag ccctcctaga tgtgatactt     720

cccttcatct tcctattaga gggtagttat ctcccccttt tagccttcgt gatgttcgaa     780

gtaataacca tggtgatagc ggtaatctgg gagggaggct ccttaatcga ggccctgctt     840

ttccccgttg tcctctggtt cttggccctc ttctaccttt ccctccactt atacggatac     900

gctgtctatg gcttgaaaag tttgcttaga ttcactcgtt cctag                    945

<210>  2
<211>  314
<212>  PRT
<213>  Pyrococcus horikoshii

<400>  2

Met Ser Ile Ile Ile Pro Ala Tyr Asn Glu Glu Glu Asn Ile Lys Arg
1               5                   10                  15

Ala Ile Lys Ala Ala Leu Ser Gln Asp Tyr Pro Val Ser Glu Val Ile
            20                  25                  30

Val Val Asp Asp Gly Ser Glu Asp Gly Thr Tyr Glu Arg Ala Lys Glu
        35                  40                  45

Ile Lys Asp His Arg Leu Lys Val Leu Lys Ile Asp His Arg Gly Lys
    50                  55                  60

Ala Gly Ala Ile Asn Glu Gly Leu Lys Ile Ala Lys Gly Ser Val Ile
65                  70                  75                  80

Val Thr Thr Asp Ala Asp Ser Phe Met Ser Lys Asp Ala Val Arg Arg
                85                  90                  95

Leu Val Glu Arg Phe Tyr Ser Asp Asp Val Val Ala Val Gly Gly Gln
            100                 105                 110

Ile Arg Val Ile Val Glu Ser Phe Met Thr Leu Val Gln Asp Ile Glu
        115                 120                 125

His Leu Arg Ile Ala Met Tyr Arg Arg Gly Lys Glu Leu Glu Asn Leu
    130                 135                 140

Ser Leu Ala Pro Gly Pro Leu Ser Ala Phe Arg Arg Asp Val Leu Glu
145                 150                 155                 160

Lys Ile Gly Gly Ile Gln Arg Ser Ile Val Glu Asp Tyr Ala Thr Thr
                165                 170                 175

Lys Ile Val Lys Lys Tyr Gly Arg Val Val Tyr Ala Pro Lys Ala Lys
            180                 185                 190

Leu Tyr Thr Arg Met Pro Ile Thr Leu Arg Asp Leu Trp Arg Gln Arg
        195                 200                 205

Lys Arg Trp Phe Leu Gly Asp Leu Gln His His Asn Leu Asn Asp Leu
    210                 215                 220

Leu Asn Leu Leu Leu Gly Asp Val Ile Ala Leu Leu Asp Val Ile Leu
225                 230                 235                 240

Pro Phe Ile Phe Leu Leu Glu Gly Ser Tyr Leu Pro Leu Leu Ala Phe

245                 250                 255

Val Met Phe Glu Val Ile Thr Met Val Ile Ala Val Ile Trp Glu Gly
            260                 265                 270

Gly Ser Leu Ile Glu Ala Leu Leu Phe Pro Val Val Leu Trp Phe Leu
        275                 280                 285

Ala Leu Phe Tyr Leu Ser Leu His Leu Tyr Gly Tyr Ala Val Tyr Gly
    290                 295                 300

Leu Lys Ser Leu Leu Arg Phe Thr Arg Ser
305                 310

<210>  3
<211>  4127
<212>  DNA
<213>  Unknown

<220>
<223>  Artificial sequence

<400>  3
aagaaaccaa ttgtccatat tgcatcagac attgccgtca ctgcgtcttt tactggctct      60

tctcgctaac caaaccggta accccgctta ttaaaagcat tctgtaacaa agcgggacca     120

aagccatgac aaaaacgcgt aacaaaagtg tctataatca cggcagaaaa gtccacattg     180

attatttgca cggcgtcaca ctttgctatg ccatagcatt tttatccata agattagcgg     240

atcctacctg acgcttttta tcgcaactct ctactgtttc tccatacccg tttttttggg     300

ctagaaataa ttttgtttaa ctttaagaag gagatataca tacccatggg ctctggatcc     360

ggtgatgacg atgacaagct cgcccttaag ggcgagcttg aaggtaagcc tatccctaac     420

cctctcctcg gtctcgattc tacgcgtacc ggtcatcatc accatcacca ttgagtttaa     480

acggtctcca gcttggctgt tttggcggat gagagaagat tttcagcctg atacagatta     540

aatcagaacg cagaagcggt ctgataaaac agaatttgcc tggcggcagt agcgcggtgg     600

tcccacctga ccccatgccg aactcagaag tgaaacgccg tagcgccgat ggtagtgtgg     660

ggtctcccca tgcgagagta gggaactgcc aggcatcaaa taaaacgaaa ggctcagtcg     720

aaagactggg cctttcgttt tatctgttgt ttgtcggtga acgctctcct gagtaggaca     780

aatccgccgg gagcggattt gaacgttgcg aagcaacggc ccggagggtg gcgggcagga     840

cgcccgccat aaactgccag gcatcaaatt aagcagaagg ccatcctgac ggatggcctt     900

tttgcgtttc tacaaactct ttttgtttat ttttctaaat acattcaaat atgtatccgc     960

tcatgagaca ataaccctga taaatgcttc aataatattg aaaaaggaag agtatgagta    1020

ttcaacattt ccgtgtcgcc cttattccct tttttgcggc attttgcctt cctgtttttg    1080

ctcacccaga aacgctggtg aaagtaaaag atgctgaaga tcagttgggt gcacgagtgg 1140

gttacatcga actggatctc aacagcggta agatccttga gagttttcgc cccgaagaac 1200

gttttccaat gatgagcact tttaaagttc tgctatgtgg cgcggtatta tcccgtgttg 1260

acgccgggca agagcaactc ggtcgccgca tacactattc tcagaatgac ttggttgagt 1320

actcaccagt cacagaaaag catcttacgg atggcatgac agtaagagaa ttatgcagtg 1380

ctgccataac catgagtgat aacactgcgg ccaacttact tctgacaacg atcggaggac 1440

cgaaggagct aaccgctttt ttgcacaaca tgggggatca tgtaactcgc cttgatcgtt 1500

gggaaccgga gctgaatgaa gccataccaa acgacgagcg tgacaccacg atgcctgtag 1560

caatggcaac aacgttgcgc aaactattaa ctggcgaact acttactcta gcttcccggc 1620

aacaattaat agactggatg gaggcggata aagttgcagg accacttctg cgctcggccc 1680

ttccggctgg ctggtttatt gctgataaat ctggagccgg tgagcgtggg tctcgcggta 1740

tcattgcagc actggggcca gatggtaagc cctcccgtat cgtagttatc tacacgacgg 1800

ggagtcaggc aactatggat gaacgaaata gacagatcgc tgagataggt gcctcactga 1860

ttaagcattg gtaactgtca gaccaagttt actcatatat actttagatt gatttaaaac 1920

ttcattttta atttaaaagg atctaggtga agatcctttt tgataatctc atgaccaaaa 1980

tcccttaacg tgagttttcg ttccactgag cgtcagaccc cgtagaaaag atcaaaggat 2040

cttcttgaga tccttttttt ctgcgcgtaa tctgctgctt gcaaacaaaa aaaccaccgc 2100

taccagcggt ggtttgtttg ccggatcaag agctaccaac tctttttccg aaggtaactg 2160

gcttcagcag agcgcagata ccaaatactg tccttctagt gtagccgtag ttaggccacc 2220

acttcaagaa ctctgtagca ccgcctacat acctcgctct gctaatcctg ttaccagtgg 2280

ctgctgccag tggcgataag tcgtgtctta ccgggttgga ctcaagacga tagttaccgg 2340

ataaggcgca gcggtcgggc tgaacggggg gttcgtgcac acagcccagc ttggagcgaa 2400

cgacctacac cgaactgaga tacctacagc gtgagctatg agaaagcgcc acgcttcccg 2460

aagggagaaa ggcggacagg tatccggtaa gcggcagggt cggaacagga gagcgcacga 2520

gggagcttcc agggggaaac gcctggtatc tttatagtcc tgtcgggttt cgccacctct 2580

gacttgagcg tcgatttttg tgatgctcgt caggggggcg gagcctatgg aaaaacgcca 2640

gcaacgcggc ctttttacgg ttcctggcct tttgctggcc ttttgctcac atgttctttc 2700

ctgcgttatc ccctgattct gtggataacc gtattaccgc ctttgagtga gctgataccg 2760

ctcgccgcag ccgaacgacc gagcgcagcg agtcagtgag cgaggaagcg gaagagcgcc 2820

tgatgcggta ttttctcctt acgcatctgt gcggtatttc acaccgcata tggtgcactc 2880

tcagtacaat ctgctctgat gccgcatagt taagccagta tacactccgc tatcgctacg 2940

tgactgggtc atggctgcgc cccgacaccc gccaacaccc gctgacgcgc cctgacgggc 3000

ttgtctgctc ccggcatccg cttacagaca agctgtgacc gtctccggga gctgcatgtg 3060

tcagaggttt tcaccgtcat caccgaaacg cgcgaggcag cagatcaatt cgcgcgcgaa 3120

ggcgaagcgg catgcataat gtgcctgtca aatggacgaa gcagggattc tgcaaaccct 3180

atgctactcc gtcaagccgt caattgtctg attcgttacc aattatgaca acttgacggc 3240

tacatcattc actttttctt cacaaccggc acggaactcg ctcgggctgg ccccggtgca 3300

ttttttaaat acccgcgaga aatagagttg atcgtcaaaa ccaacattgc gaccgacggt 3360

ggcgataggc atccgggtgg tgctcaaaag cagcttcgcc tggctgatac gttggtcctc 3420

gcgccagctt aagacgctaa tccctaactg ctggcggaaa agatgtgaca gacgcgacgg 3480

cgacaagcaa acatgctgtg cgacgctggc gatatcaaaa ttgctgtctg ccaggtgatc 3540

gctgatgtac tgacaagcct cgcgtacccg attatccatc ggtggatgga gcgactcgtt 3600

aatcgcttcc atgcgccgca gtaacaattg ctcaagcaga tttatcgcca gcagctccga 3660

atagcgccct tccccttgcc cggcgttaat gatttgccca aacaggtcgc tgaaatgcgg 3720

ctggtgcgct tcatccgggc gaaagaaccc cgtattggca aatattgacg gccagttaag 3780

ccattcatgc cagtaggcgc gcggacgaaa gtaaacccac tggtgatacc attcgcgagc 3840

ctccggatga cgaccgtagt gatgaatctc tcctggcggg aacagcaaaa tatcacccgg 3900

tcggcaaaca aattctcgtc cctgattttt caccaccccc tgaccgcgaa tggtgagatt 3960

gagaatataa cctttcattc ccagcggtcg gtcgataaaa aaatcgagat aaccgttggc 4020

ctcaatcggc gttaaacccg ccaccagatg ggcattaaac gagtatcccg gcagcagggg 4080

atcattttgc gcttcagcca tacttttcat actcccgcca ttcagag 4127

<210> 4
<211> 6315
<212> DNA
<213> Unknown

<220>
<223> Artificial sequence

<400> 4
caaggagatg gcgcccaaca gtcccccggc cacggggcct gccaccatac ccacgccgaa 60

acaagcgctc atgagcccga agtggcgagc ccgatcttcc ccatcggtga tgtcggcgat 120

ataggcgcca gcaaccgcac ctgtggcgcc ggtgatgccg gccacgatgc gtccggcgta 180

gaggatcgag atctcgatcc cgcgaaatta atacgactca ctatagggga attgtgagcg 240

gataacaatt cccctctaga aataattttg tttaacttta agaaggagat atacataatg 300

ggatctgata aaattattca tctgactgat gattcttttg atactgatgt acttaaggca 360

gatggtgcaa tcctggttga tttctgggca cactggtgcg gtccgtgcaa aatgatcgct 420

ccgattctgg atgaaatcgc tgacgaatat cagggcaaac tgaccgttgc aaaactgaac 480

atcgatcaca acccgggcac tgcgccgaaa tatggcatcc gtggtatccc gactctgctg 540

ctgttcaaaa acggtgaagt ggcggcaacc aaagtgggtg cactgtctaa aggtcagttg 600

aaagagttcc tcgacgctaa cctggccggc tctggatccg gtgatgacga tgacaagctg 660

ggaattgatc ccttcaccaa gggcgagctc aagcttgaag gtaagcctat ccctaaccct 720

ctcctcggtc tcgattctac gcgtaccggt catcatcacc atcaccattg agtttgatcc 780

ggctgctaac aaagcccgaa aggaagctga gttggctgct gccaccgctg agcaataact 840

agcataaccc cttggggcct ctaaacgggt cttgaggggt tttttgctga aaggaggaac 900

tatatccgga tatccacagg acgggtgtgg tcgccatgat cgcgtagtcg atagtggctc 960

caagtagcga agcgagcagg actgggcggc ggccaaagcg gtcggacagt gctccgagaa 1020

cgggtgcgca tagaaattgc atcaacgcat atagcgctag cagcacgcca tagtgactgg 1080

cgatgctgtc ggaatggacg atatcccgca agaggcccgg cagtaccggc ataaccaagc 1140

ctatgcctac agcatccagg gtgacggtgc cgaggatgac gatgagcgca ttgttagatt 1200

tcatacacgg tgcctgactg cgttagcaat ttaactgtga taaactaccg cattaaagct 1260

agcttatcga tgataagctg tcaaacatga gaattaattc ttgaagacga aagggcctcg 1320

tgatacgcct atttttatag gttaatgtca tgataataat ggtttcttag acgtcaggtg 1380

gcacttttcg gggaaatgtg cgcggaaccc ctatttgttt atttttctaa atacattcaa 1440

atatgtatcc gctcatgaga caataaccct gataaatgct tcaataatat tgaaaaagga 1500

agagtatgag tattcaacat ttccgtgtcg cccttattcc cttttttgcg gcattttgcc 1560

ttcctgtttt tgctcaccca gaaacgctgg tgaaagtaaa agatgctgaa gatcagttgg 1620

gtgcacgagt gggttacatc gaactggatc tcaacagcgg taagatcctt gagagttttc 1680

gccccgaaga acgttttcca atgatgagca cttttaaagt tctgctatgt ggcgcggtat 1740

tatcccgtgt tgacgccggg caagagcaac tcggtcgccg catacactat tctcagaatg 1800

acttggttga gtactcacca gtcacagaaa agcatcttac ggatggcatg acagtaagag 1860

aattatgcag tgctgccata accatgagtg ataacactgc ggccaactta cttctgacaa 1920

cgatcggagg accgaaggag ctaaccgctt ttttgcacaa catgggggat catgtaactc 1980

gccttgatcg ttgggaaccg gagctgaatg aagccatacc aaacgacgag cgtgacacca 2040

cgatgcctgc agcaatggca acaacgttgc gcaaactatt aactggcgaa ctacttactc 2100

tagcttcccg gcaacaatta atagactgga tggaggcgga taaagttgca ggaccacttc 2160

tgcgctcggc ccttccggct ggctggttta ttgctgataa atctggagcc ggtgagcgtg 2220

ggtctcgcgg tatcattgca gcactggggc cagatggtaa gccctcccgt atcgtagtta 2280

tctacacgac ggggagtcag gcaactatgg atgaacgaaa tagacagatc gctgagatag 2340

gtgcctcact gattaagcat tggtaactgt cagaccaagt ttactcatat atactttaga 2400

ttgatttaaa acttcatttt taatttaaaa ggatctaggt gaagatcctt tttgataatc 2460

tcatgaccaa aatcccttaa cgtgagtttt cgttccactg agcgtcagac cccgtagaaa 2520

agatcaaagg atcttcttga gatccttttt ttctgcgcgt aatctgctgc ttgcaaacaa 2580

aaaaaccacc gctaccagcg gtggtttgtt tgccggatca agagctacca actctttttc 2640

cgaaggtaac tggcttcagc agagcgcaga taccaaatac tgtccttcta gtgtagccgt 2700

agttaggcca ccacttcaag aactctgtag caccgcctac atacctcgct ctgctaatcc 2760

tgttaccagt ggctgctgcc agtggcgata agtcgtgtct taccgggttg gactcaagac 2820

gatagttacc ggataaggcg cagcggtcgg gctgaacggg gggttcgtgc acacagccca 2880

gcttggagcg aacgacctac accgaactga gatacctaca gcgtgagcta tgagaaagcg 2940

ccacgcttcc cgaagggaga aaggcggaca ggtatccggt aagcggcagg gtcggaacag 3000

gagagcgcac gagggagctt ccagggggaa acgcctggta tctttatagt cctgtcgggt 3060

ttcgccacct ctgacttgag cgtcgatttt tgtgatgctc gtcagggggg cggagcctat 3120

ggaaaaacgc cagcaacgcg gcctttttac ggttcctggc cttttgctgg ccttttgctc 3180

acatgttctt tcctgcgtta tcccctgatt ctgtggataa ccgtattacc gcctttgagt 3240

gagctgatac cgctcgccgc agccgaacga ccgagcgcag cgagtcagtg agcgaggaag 3300

cggaagagcg cctgatgcgg tattttctcc ttacgcatct gtgcggtatt tcacaccgca 3360

atggtgcact ctcagtacaa tctgctctga tgccgcatag ttaagccagt atacactccg 3420

ctatcgctac gtgactgggt catggctgcg ccccgacacc cgccaacacc cgctgacgcg 3480

ccctgacggg cttgtctgct cccggcatcc gcttacagac aagctgtgac cgtctccggg 3540

agctgcatgt gtcagaggtt ttcaccgtca tcaccgaaac gcgcgaggca gctgcggtaa 3600

agctcatcag cgtggtcgtg aagcgattca cagatgtctg cctgttcatc cgcgtccagc 3660

tcgttgagtt tctccagaag cgttaatgtc tggcttctga taaagcgggc catgttaagg 3720

gcggtttttt cctgtttggt cactgatgcc tccgtgtaag ggggatttct gttcatgggg 3780

gtaatgatac cgatgaaacg agagaggatg ctcacgatac gggttactga tgatgaacat 3840

gcccggttac tggaacgttg tgagggtaaa caactggcgg tatggatgcg gcgggaccag 3900

agaaaaatca ctcagggtca atgccagcgc ttcgttaata cagatgtagg tgttccacag 3960

ggtagccagc agcatcctgc gatgcagatc cggaacataa tggtgcaggg cgctgacttc 4020

cgcgtttcca gactttacga aacacggaaa ccgaagacca ttcatgttgt tgctcaggtc 4080

gcagacgttt tgcagcagca gtcgcttcac gttcgctcgc gtatcggtga ttcattctgc 4140

taaccagtaa ggcaaccccg ccagcctagc cgggtcctca acgacaggag cacgatcatg 4200

cgcacccgtg gccaggaccc aacgctgccc gagatgcgcc gcgtgcggct gctggagatg 4260

gcggacgcga tggatatgtt ctgccaaggg ttggtttgcg cattcacagt tctccgcaag 4320

aattgattgg ctccaattct tggagtggtg aatccgttag cgaggtgccg ccggcttcca 4380

ttcaggtcga ggtggcccgg ctccatgcac cgcgacgcaa cgcggggagg cagacaaggt 4440

atagggcggc gcctacaatc catgccaacc cgttccatgt gctcgccgag gcggcataaa 4500

tcgccgtgac gatcagcggt ccaatgatcg aagttaggct ggtaagagcc gcgagcgatc 4560

cttgaagctg tccctgatgg tcgtcatcta cctgcctgga cagcatggcc tgcaacgcgg 4620

gcatcccgat gccgccggaa gcgagaagaa tcataatggg gaaggccatc cagcctcgcg 4680

tcgcgaacgc cagcaagacg tagcccagcg cgtcggccgc catgccggcg ataatggcct 4740

gcttctcgcc gaaacgtttg gtggcgggac cagtgacgaa ggcttgagcg agggcgtgca 4800

agattccgaa taccgcaagc gacaggccga tcatcgtcgc gctccagcga aagcggtcct 4860

cgccgaaaat gacccagagc gctgccggca cctgtcctac gagttgcatg ataaagaaga 4920

cagtcataag tgcggcgacg atagtcatgc cccgcgccca ccggaaggag ctgactgggt 4980

tgaaggctct caagggcatc ggtcgagatc ccggtgccta atgagtgagc taacttacat 5040

taattgcgtt gcgctcactg cccgctttcc agtcgggaaa cctgtcgtgc cagctgcatt 5100

aatgaatcgg ccaacgcgcg gggagaggcg gtttgcgtat tgggcgccag ggtggttttt 5160

cttttcacca gtgagacggg caacagctga ttgcccttca ccgcctggcc ctgagagagt 5220

tgcagcaagc ggtccacgct ggtttgcccc agcaggcgaa aatcctgttt gatggtggtt 5280

aacggcggga tataacatga gctgtcttcg gtatcgtcgt atcccactac cgagatatcc 5340

gcaccaacgc gcagcccgga ctcggtaatg gcgcgcattg cgcccagcgc catctgatcg 5400

ttggcaacca gcatcgcagt gggaacgatg ccctcattca gcatttgcat ggtttgttga 5460

aaaccggaca tggcactcca gtcgccttcc cgttccgcta tcggctgaat ttgattgcga 5520

gtgagatatt tatgccagcc agccagacgc agacgcgccg agacagaact taatgggccc 5580

gctaacagcg cgatttgctg gtgacccaat gcgaccagat gctccacgcc cagtcgcgta 5640

ccgtcttcat gggagaaaat aatactgttg atgggtgtct ggtcagagac atcaagaaat 5700

aacgccggaa cattagtgca ggcagcttcc acagcaatgg catcctggtc atccagcgga 5760

tagttaatga tcagcccact gacgcgttgc gcgagaagat tgtgcaccgc cgctttacag 5820

gcttcgacgc cgcttcgttc taccatcgac accaccacgc tggcacccag ttgatcggcg 5880

cgagatttaa tcgccgcgac aatttgcgac ggcgcgtgca gggccagact ggaggtggca 5940

acgccaatca gcaacgactg tttgcccgcc agttgttgtg ccacgcggtt gggaatgtaa 6000

ttcagctccg ccatcgccgc ttccactttt tcccgcgttt tcgcagaaac gtggctggcc 6060

tggttcacca cgcgggaaac ggtctgataa gagacaccgg catactctgc gacatcgtat 6120

aacgttactg gtttcacatt caccaccctg aattgactct cttccgggcg ctatcatgcc 6180

ataccgcgaa aggttttgcg ccattcgatg gtgtccggga tctcgacgct ctcccttatg 6240

cgactcctgc attaggaagc agcccagtag taggttgagg ccgttgagca ccgccgccgc 6300

aaggaatggt gcatg 6315

<210> 5
<211> 20
<212> DNA
<213> Unknown

<220>
<223> Primer

<400> 5
gtgagcataa taatcccagc 20

<210> 6
<211> 22
<212> DNA
<213> Unknown

<220>
<223> Primer

<400> 6
ctaggaacga gtgaatctaa gc 22

<210> 7
<211> 20
<212> DNA
<213> Unknown

<220>
<223> Primer

<400> 7
atgccatagc atttttatcc 20

<210> 8
<211> 18
<212> DNA
<213> Unknown

<220>
<223> Primer

<400> 8
gatttaatct gtatcagg 18

<210> 9
<211> 35
<212> DNA
<213> Unknown

<220>
<223>  Primer

<400>  9
ggagatatac atacccatga aaatcaccat ttccg                                35


<210>  10
<211>  21
<212>  DNA
<213>  Unknown

<220>
<223>  Primer

<400>  10
ttagtcgctg ccaaagagat c                                               21


<210>  11
<211>  34
<212>  DNA
<213>  Unknown

<220>
<223>  Primer

<400>  11
ggagatatac atacccatgg ctgccattaa tacg                                 34


<210>  12
<211>  24
<212>  DNA
<213>  Unknown

<220>
<223>  Primer

<400>  12
ttacttctta atgcccatct cttc                                            24


<210>  13
<211>  43
<212>  DNA
<213>  Unknown

<220>
<223>  Primer

<400>  13
tcaccatcac cattgagttt ttaagaagga gatatacata ccc                       43


<210>  14
<211>  38
<212>  DNA
<213>  Unknown

<220>
<223>  Primer

<400>  14
agccaagctg gagaccgttt ttagtcgctg ccaaagag                              38


<210>  15
<211>  28
<212>  DNA
<213>  Unknown

<220>
<223>  Primer

<400>  15
ccttcttaaa ttagtcgctg ccaaagag                                         28


<210>  16
<211>  33
<212>  DNA
<213>  Unknown

<220>
<223>  Primer

<400>  16
cagcgactaa tttaagaagg agatatacat acc                                   33


<210>  17
<211>  39
<212>  DNA
<213>  Unknown

<220>
<223>  Primer

<400>  17
agccaagctg gagaccgttt ttacttctta atgcccatc                             39


<210>  18
<211>  18
<212>  DNA
<213>  Unknown

<220>
<223>  Primer

<400>  18
aacggtctcc agcttggc                                                    18


<210>  19
<211>  18
<212>  DNA
<213>  Unknown

<220>
<223>  Primer

<400>  19
taaactcaat ggtgatgg                                                    18

<210>  20
<211>  42
<212>  DNA
<213>  Unknown

<220>
<223>  Primer

<400>  20
caccatcacc attgagttta taagaaggag atatacatac cc                          42


<210>  21
<211>  38
<212>  DNA
<213>  Unknown

<220>
<223>  Primer

<400>  21
cagccaagct ggagaccgtt ttagtcgctg ccaaagag                               38


<210>  22
<211>  43
<212>  DNA
<213>  Unknown

<220>
<223>  Primer

<400>  22
tcaccatcac cattgagttt ttaagaagga gatatacata ccc                         43


<210>  23
<211>  38
<212>  DNA
<213>  Unknown

<220>
<223>  Primer

<400>  23
agccaagctg gagaccgttt ttagtcgctg ccaaagag                               38


<210>  24
<211>  24
<212>  DNA
<213>  Unknown

<220>
<223>  Primer

<400>  24
caccgtgagc ataataatcc cagc                                              24


<210>  25

<211>  22
<212>  DNA
<213>  Unknown

<220>
<223>  Primer

<400>  25
ctaggaacga gtgaatctaa gc                                              22


<210>  26
<211>  876
<212>  DNA
<213>  Unknown

<220>
<223>  Artificial sequence

<400>  26
gctatgccat agcattttta tccataagat tagcggatcc tacctgacgc tttttatcgc      60

aactctctac tgtttctcca tacccgtttt tttgggctag aaataatttt gtttaacttt     120

aagaaggaga tatacatacc catgggctct ggatccggtg atgacgatga caagctcgcc     180

cttaagggcg agcttgaagg taagcctatc cctaaccctc tcctcggtct cgattctacg     240

cgtaccggtc atcatcacca tcaccattga gtttaaacgg tctccagctt ggctgttttg     300

gcggatgaga gaagattttc agcctgatac agattaaatc agaacgcaga agcggtctga     360

taaaacagaa tttgcctggc ggcagtagcg cggtggtccc acctgacccc atgccgaact     420

cagaagtgaa acgccgtagc gccgatggta gtgtggggtc tccccatgcg agagtaggga     480

actgccaggc atcaaataaa acgaaaggct cagtcgaaag actgggcctt tcgttttatc     540

tgttgtttgt cggtgaacgc tctcctgagt aggacaaatc cgccgggagc ggatttgaac     600

gttgcgaagc aacggcccgg agggtggcgg gcaggacgcc cgccataaac tgccaggcat     660

caaattaagc agaaggccat cctgacggat ggcctttttg cgtttctaca aactcttttt     720

gtttattttt ctaaatacat tcaaatatgt atccgctcat gagacaataa ccctgataaa     780

tgcttcaata atattgaaaa aggaagagta tgagtattca acatttccgt gtcgccctta     840

ttcccttttt tgcggcattt tgccttcctg tttttg                               876


<210>  27
<211>  659
<212>  DNA
<213>  Unknown

<220>
<223>  Artificial sequence

<400>  27
ataggcgcca gcaaccgcac ctgtggcgcc ggtgatgccg gccacgatgc gtccggcgta      60

gaggatcgag atctcgatcc cgcgaaatta atacgactca ctatagggga attgtgagcg     120

gataacaatt cccctctaga aataattttg tttaacttta agaaggagat atacataatg 180

ggatctgata aaattattca tctgactgat gattcttttg atactgatgt acttaaggca 240

gatggtgcaa tcctggttga tttctgggca cactggtgcg gtccgtgcaa aatgatcgct 300

ccgattctgg atgaaatcgc tgacgaatat cagggcaaac tgaccgttgc aaaactgaac 360

atcgatcaca acccgggcac tgcgccgaaa tatggcatcc gtggtatccc gactctgctg 420

ctgttcaaaa acggtgaagt ggcggcaacc aaagtgggtg cactgtctaa aggtcagttg 480

aaagagttcc tcgacgctaa cctggccggc tctggatccg gtgatgacga tgacaagctg 540

ggaattgatc ccttcaccaa gggcgagctc aagcttgaag taagcctatc cctaaccctc 600

tcctcggtct cgattctacg cgtaccggtc atcatcacca tcaccattga gtttgatcc 659

## Description

### Technical field

The present invention refers to an isolated gene coding for a Glycosyl transferase 2 enzyme, to the isolated enzyme Glycosyl transferases 2 encoded by this gene, and to a process for producing sulfated glycosaminoglycans (GAGs).

Therefore, the present invention has utility in pharmaceuticals, cosmetics, nutraceuticals, and food fields.

In the description below, the references into brackets ([ ]) refer to the listing of references situated at the end of the text.

### Background of the Invention

Glycosaminoglycans (GAGs), also known as mucopolysaccharides, are long-chain, unbranched polysaccharides that are found on the cell surface and constitute a major constituents of the extracellular matrix. They are negatively-charged polysaccharide of diverse molecular weights, composed of repeating disaccharide units that consist of either sulfated or non-sulfated monosaccharides. They contain an amino sugar, either glucose based or galactose based (GlcNAc or GalNAc (N-acetylgalactosamine) or α or β D-glucosamine) and an uronic acid (either glucuronic acid and/or α-L-iduronic acid, a C5 epimer of GlcA) except for keratan which does not contain an uronic acid and is composed of an amino sugar and galactose disaccharides. Based on the composition of the disaccharide unit of each GAG, they are classified into six groups: chondroitin sulfate (CS), keratan sulfate (KS), hyaluronic acid also called hyaluronan (HA), dermatan sulfate (DS), heparin and heparan sulfate (HS). They are all sulfated at different positions except for HA. Moreover, the molecular size and the sulfation type of GAGs vary depending on the tissue, and their state either as part of proteoglycan or as free chains. In this regard, glycosaminoglycans play important roles in physiological and pathological conditions.

In vertebrates, all these GAGs are present, where three major classes predominate including (1) heparin and heparin sulfate (HS) that are both composed of alternating 4-linked uronic acid and 4-linked α-GlcNAC but differ only in the relative proportions of their monosaccharide and disaccharide substructures units, (2) hyaluronan (HA) and (3) chondroitin sulfate (CS) and dermatan sulfate (DS). All these GAGs, except for HA, acquire structural variability by modifications that involve sulfation and uronate epimerization, which are the basis for the wide variety of domain structures with biological activities.

In addition, the structural variations of GAGs and their heterogeneities regarding their sulfation content (except HA) and their common occurrence at the extracellular matrix (ECM) or at the surface of cells, are related to their role as structural components and regulators of a variety of functions of proteins, cells and tissues in the human body and are all contributing factors to the diversity of their biomedical roles by their ability to bind to multiple extracellular proteins whose actions are spread in various pathophysiological events, as inflammation, cancer, sugar metabolism, bone remodeling and resorption, tissue development, regeneration and repair, and wound repair and coagulation.

All these data investigate the biological importance of GAGs, notably the hyaluronan (HA) and chondroitin sulfate (CS) that represent the top ranked products in industrial biotechnology for biomedical applications, but also in the fields of cosmetics, nutraceuticals and food. The world market of these GAGs is growing to 1 billion dollars per year.

Most of these industrial GAGs have been extracted from animal sources. However, the use of animal-derived biopolymers as human therapeutics has faced considerable safety concerns due to the risk of carrying contaminations. Given the high demand and great biological and therapeutic functions of GAGs, especially in the pharmaceutical sector, a remarkable progress has been made in the development of genetically engineered endotoxin-free microoorganisms such as *Escherichia coli* for their production. Indeed, GAGs, especially hyaluronan (HA) and chondroitin have been recently produced by metabolic engineering strategy by which their synthases, hyaluronan synthase and chondroitin polymerase respectively, have been cloned and expressed in *E*. *coli* (Yu, H. and G. Stephanopoulos, Metabolic engineering of Escherichia coli for biosynthesis of hyaluronic acid. Metab Eng, 2008. 10(1): p. 24-32 ([1]); He W, Fu L, Li G, Andrew Jones J, Linhardt RJ, Koffas M. Production of chondroitin in metabolically engineered E. coli. Metab Eng. 2015;27:92-100 ([2])).

Glycosyl transferases (GTs) are enzymes that catalyze the biosynthesis of disaccharides, oligosaccharides, polysaccharides and glycoconjugates by the transfer of a sugar residue from activated donor molecules to appropriate acceptor molecules to form new glycosidic linkages. They play essential roles in fundamental biological processes and can be used for the synthesis of many high valuable natural products and might be exploited for novel medical applications.

Currently, GTs are classified into 110 families and their number is continuously increasing by discovering new GT genes with the evolution of gene database sequences. The study of these enzymes is indispensable for their exploitation in order to develop their roles in biotechnology, in particular for producing GAGs by metabolic engineering strategy.

Although a wide range of GTs have been cloned and well-characterized, a need exists of alternative biotechnological process and tools for producing GAGs, especially novel recombinant GAGs.

### Description of the invention

The present invention fulfills these and other needs.

Surprisingly, the Applicants discovered novel GTs from hyperthermophilic Archaea, more specifically the GT-2 family. Interestingly, they found that these thermostable enzymes are able to catalyze the polymerization of a novel GAG polysaccharide displaying unique properties which give them remarkable industrial relevance.

GTs from hyperthermophilic Archaea are interesting for their resistance to heat and denaturants and their remarkable stability which give them an industrial relevance. While GTs from many organisms have been characterized, few examples of GTs from Archaea are described particularly for the large processive GTs. These enzymes are involved in the synthesis of carbohydrate polymers such as chitin, alginate, poly-*N-*acetylglucosamine (PNAG) and some GAGs such as hyaluronan and chondroitin that play a major role in different tissues and organs and have numerous applications in the fields of pharmaceuticals, cosmetics, nutraceuticals, and foods.

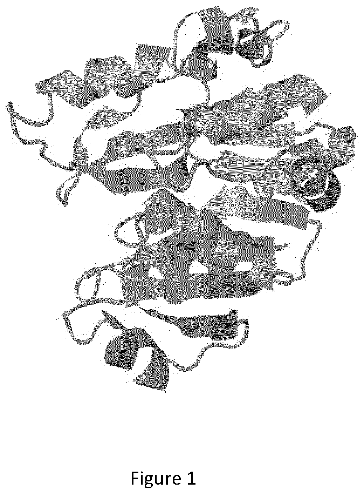

Based on a bioinformatic study, a novel family from the GT2-A processive enzymes was detected in Archaeal sequenced genomes and members of this family show the characteristic GT-A fold with two Rossmann fold-like domains (Figure 1). This gene family is not distributed in all Archaea. It is ubiquitous to most of the thermococcales group and some halophiles. Phylogenetic analysis of the gene family showed that it forms a separate cluster from the rest of the analyzed GT2-A families. Search for homologs and analysis of domain architecture revealed that the closest homologs to this gene family are the prokaryotic hyaluronan synthase and prokaryotic chondroitin polymerase. Among this family, the Applicants have selected a protein composed of 314 amino acids from the hyperthermophilic Archaeon *Pyrococcus horikoshii.*

Accordingly, in a first aspect, the present invention provides an isolated gene coding for the enzyme Glycosyl transferase 2 from *Pyrococcus horikoshii* OT3, the nucleotide sequence of said gene comprising or consisting of sequence SEQ ID NO: 1, or its homologs from hyperthermophilic *Archaea.*

In another aspect, the present invention provides an isolated enzyme Glycosyl transferases 2, encoded by the isolated gene defined above. Advantageously, the isolated enzyme comprises or consists to the sequence SEQ ID NO: 2, or an homologous sequence from hyperthermophilic *Archaea.*

As used herein, the term " glycosyl transferase 2 " refers to an enzyme having glycosyl transferase activity, which catalyzes the biosynthesis of disaccharides, oligosaccharides, polysaccharides and/or glycoconjugates by the transfer of a sugar residue from activated donor molecules to appropriate acceptor molecules to form new glycosidic linkages.

As used herein, the term "gene" refers to a segment of DNA involved in producing a polypeptide, that may or may not include regions preceding and following the coding region, e.g. 5' untranslated (51 UTR) or "leader" sequences and 3' UTR or "trailer" sequences, as well as intervening sequences (introns) between individual coding segments (exons). A gene may refer to one that is naturally occurring, or a synthetic gene, as long as they keep the glycosyl transferase activity.

As used herein, the terms "isolated gene" refer to a nucleic acid or a polypeptide that is removed from at least one other material or component with which it is naturally associated as found in nature. The term « isolated » is synonym to « separated » or « purified ». An isolated polynucleotide, thereof, includes, but is not limited to, a culture broth containing the polynucleotide inserted in a heterologous host cell, or to a culture broth containing secreted polypeptide expressed in a cell, for example an heterologous host cell.

The techniques used to isolate or clone a polynucleotide encoding a polypeptide are known in the art, including isolation from genomic DNA, preparation from cDNA, or a combination thereof. The cloning of the polynucleotides of the present disclosure from such genomic DNA can be effected, e.g., by using the polymerase chain reaction (PCR) or probe screening of expression libraries to detect cloned DNA fragments with shared structural features, as illustrated for example in Sambrook et al , MOLECULAR CLONING: A LABORATORY MANUAL, 2nd ed., Cold Spring Harbor, 1989, and 3rd ed., 2001 ([12]). Other nucleic acid amplification procedures such as ligase chain reaction (LCR), ligated activated transcription (LAT) and nucleotide sequence-based amplification (NASBA) may be used.

The gene or enzyme of the invention may be isolated from *Pyrococcus horikoshii* OT3 (Accession number PH_RS02140). *Pyrococcus horikoshii* is a hyperthermophilic Archaeon that belongs to the phylum Euryarchaeota within the order Thermococcales and thrive in hydrothermal vents of the deep sea. It is characterized by particular enzymes that display high thermostability allowing them to resist the elevated temperature and extreme pressure conditions giving them high resistance to denaturation and proteolysis. The properties of these enzymes have enabled them to be used in numerous biotechnological applications (Kumar, S. and R. Nussinov, How do thermophilic proteins deal with heat? Cellular and Molecular Life Sciences CMLS, 2001. 58(9): p. 1216-1233 ([3])).

Alternatively, it may be isolated from any ortholog including hyperthermophilic Archaea and halophiles, for example those listed in Table 1 below.

**Table 1 :**

| Accession number | *Organism name* |
|---|---|
| C6A0L4 | *Thermococcus sibiricus* |
| Q5JHB8 | *Thermococcus kodakarensis* |
| B6YUR6 | *Thermococcus onnurineus* |
| Q9UYC7 | *Pyrococcus abyssi* |
| 058219 | *Pyrococcus horikoshii* |
| Q8U3K1 | *Pyrococcus furiosus* |
| F4HMZ8 | *Pyrococcus sp. (strain NA2)* |
| C5A5B1 | *Thermococcus gammatolerans* |
| F8AIC0 | *Pyrococcus yayanosii* |
| F7PGN0 | *Halorhabdus tiamatea* |
| D3SRL1 | *Natrialba magadii* |
| C7NQ00 | *Halorhabdus utahensis* |
| Q8TN31 | *Methanosarcina acetivorans* |
| Q46B82 | *Methanosarcina barkeri* |
| B9LWG0 | *Halorubrum lacusprofundi* |
| D3SVK8 | *Natrialba magadii* |
| E4NTD4 | *Halogeometricum borinquense* |
| C5A6A1 | *Thermococcus gammatolerans* |
| FOLM41 | *Thermococcus barophilus* |

As used herein, the terms "homolog" refers to DNA sequences sharing ancestry, because of either a speciation event (orthologs) or a duplication event (paralogs). Homology among DNA can also be concluded on the basis of sequence similarity or identity. Therefore, "homolog" refers also to a mutant gene having substantially the same the glycosyl transferase activity as GT2 from hyperthermophilic Archaea *Pyrococcus horikoshii OT3,* and having a nucleotide sequence that is at least about 80% identical, or at least about 85% identical, or at least about 90% identical, or at least about 91% identical, or at least about 92% identical, or at least about 93% identical, or at least about 94% identical, or at least about 95% identical, or at least about 96% identical, or at least about 97% identical, or at least about 98% identical, at least about 99% identical, to SEQ ID NO:1 or any of the homologs of table 1.

The nucleotide sequence of SEQ ID NO: 1, or subsequence thereof, as well as the amino acid sequence of SEQ ID NO:2, or fragments thereof, may be used to design a nucleic acid probe to identify and clone DNA encoding polypeptides having glycosyl transferase activity from strains of different genera or species according to methods well known in the art. In particular, such probes can be used for hybridization with the genomic or cDNA of the genus or species of interest, following standard southern blotting procedures, in order to identify and isolate the corresponding gene therein. Such probes can be considerably shorter than the entire sequence, but should be at least 14, preferably at least 25, more preferably at least35, and most preferably at least 70 nucleotides in length. It is, however, preferred that the nucleic acid probe is at least 100 nucleotides in length. For example, the nucleic acid probe may be at least 200 nucleotides, preferably at least 300 nucleotides, more preferably at least 400 nucleotides, or most preferably at least 500 nucleotides in length. Even longer probes may be used, e.g., nucleic acid probes which are at least 600 nucleotides, at least preferably at least 700 nucleotides, more preferably at least 800 nucleotides, or most preferably at least 900 nucleotides in length. Both DNA and RNA probes can be used. The probes are typically labeled for detecting the corresponding gene (for example, with ³²P, ³H, ³⁵S, biotin, or avidin). Such probes are encompassed by the present disclosure.

A genomic DNA or cDNA library prepared from such other organisms may, therefore, be screened for DNA which hybridizes with the probes described above and which encodes a polypeptide having alpha-glucosidase activity. Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. In order to identify a clone or DNA which is homologous with SEQ ID NO: 1, or SEQ ID NO:2, or subsequences thereof, the carrier material is used in a southern blot. For purposes of the present disclosure, hybridization indicates that the nucleotide sequence hybridizes to a labeled nucleic acid probe corresponding to the nucleotide sequence shown in SEQ ID NO: 1, or SEQ ID NO:2, their complementary strands, or subsequences thereof, under very low to very high stringency conditions. Molecules to which the nucleic acid probe hybridizes under these conditions can be detected using X-ray film.

Therefore, in some embodiments, the Glycosyl transferase 2 polypeptides of the instant disclosure are encoded by polynucleotide or nucleic acid sequences that hybridizes, for example under stringent conditions, to an isolated nucleic acid which comprises consecutive nucleotides having a sequence selected from the group consisting of the sequence SEQ ID NO: 1 or sequences from table 1, and a variant of any one having at least about 50% identity to SEQ ID NO:1, for example at least 60%, or 70%, or 80%, or 90%, or 95% or more identity to SEQ ID NO:1, or any of the homologs from table 1 under conditions of high stringency .

As used herein, the term "hybridization" refers to the process by which one strand of nucleic acid forms a duplex with, i.e., base pairs with, a complementary strand, as occurs during blot hybridization techniques and PCR techniques. Stringent hybridization conditions are exemplified by hybridization under the following conditions: 65°C and 0.1X SSC (where IX SSC = 0.15 M NaCl, 0.015 M Na₃ citrate, pH 7.0). Hybridized, duplex nucleic acids are characterized by a melting temperature (Tₘ), where one half of the hybridized nucleic acids are unpaired with the complementary strand.

Therefore, in some embodiments, the present invention includes nucleic acids that encode any recombinant or engineered enzyme having deletions, insertions, or substitutions that do not alter substantially the activity of the enzyme. These deletions, insertions, or substitutions may especially be due to the degeneracy of the genetic code, where a plurality of nucleic acids may encode the same polypeptide having the same amino acid sequence.

Another object of the invention relates to a vector, especially a plasmid vector comprising a gene coding for the enzyme Glycosyl transferase 2 from *Pyrococcus horikoshii* OT3, the nucleotide sequence of said gene comprising or consisting of the sequence SEQ ID NO : 1, or its homologs from hyperthermophilic *Archaea.*

As used herein, the term "vector" refers to any suitable vector that can be transformed into and replicated within a host cell for expressing the Glycosyl transferase 2 enzyme. Advantageously, a recombinant vector may be selected from the group comprising or consisting of a plasmid, a cosmid, a phage, an integrated cassette or a virus vector. The recombinant vector may further comprise a purified nucleic acid segment having a coding region encoding enzymatically active chondroitin sulfate synthase.

As used herein, the term "plasmid" refers to a circular double- stranded (ds) DNA construct used as a cloning vector, and which forms an extra chromosomal self-replicating genetic element in many bacteria and some eukaryotes. Vectors can be transferred to a host cell using known transformation techniques, such as those disclosed below. The plasmid may be any plasmid known in the art and adapted to the particular use according to the invention, for example pBAD-TOPO^{®} (Invitrogen^{™}) or pET102/D-TOPO^{®} (Invitrogen^{™}), pALTER-Ex1/2 (Promega), pCAL-n (Stratagene), peT series (Novagen), pGEX series (Pharmacia).

The vector may also comprises elements allowing the expression of said gene in a cell. For example, a vector comprising a nucleic acid encoding an glycosyl transferase polypeptide of the present disclosure can be transformed and replicated in a bacterial host cell as a means of propagating and amplifying the vector. The vector may also be suitably transformed into an expression host, such that the encoding polynucleotide is expressed as a functional alpha- glucosidase enzyme.

A vector useful for this purpose typically includes the components of a cloning vector, such as, for example, an element that permits autonomous replication of the vector in the selected host organism and one or more phenotypically detectable markers for selection purposes. The expression vector usually comprises control nucleotide sequences such as a promoter, operator, ribosome binding site, translation initiation signal and optionally, a repressor gene or one or more activator genes. Additionally, the expression vector may comprise a sequence coding for an amino acid sequence capable of targeting the alpha-glucosidase to a host cell organelle such as a peroxisome, or to a particular host cell compartment. For expression under the direction of control sequences, the nucleic acid sequence of the alpha-glucosidase is operably linked to the control sequences in proper manner with respect to expression.

A polynucleotide encoding a glycosyl transferase polypeptide of the present invention can be operably linked to a promoter, which allows transcription in the host cell. The promoter may be any DNA sequence that shows transcriptional activity in the host cell of choice and may be derived from genes encoding proteins either homologous or heterologous to the host cell. Examples of promoters for directing the transcription of the DNA sequence encoding a glycosyl transferase, especially in a bacterial host, include at least one promoter chosen among an arabinose inducible promoter, especially a weak arabinose inducible promoter, a T7 inducible promoter, especially a strong T7 inducible promoter, the promoter of the lac operon of E. coli, the synthetic tac promoter, the Streptomyces coelicolor agarase gene dagA or celA promoters, the promoters of the Bacillus licheniformis amylase gene (amyL), the promoters of the Bacillus stearothermophilus maltogenic amylase gene (amyM), the promoters of the Bacillus amyloliquefaciens amylase (amyQ), the promoters of the Bacillus subtilis xylA and xylB genes, and the like. Preferably, the vector is a plasmid vector comprising an arabinose inducible promoter and/or a T7 inducible promoter, operationally linked to said isolated gene.

The coding sequence can be operably linked to a signal sequence. The DNA encoding the signal sequence may be a DNA sequence naturally associated with the glycosyl transferase gene of interest to be expressed, or may be from a different genus or species as the alpha-glucosidase.

An expression vector may also comprise a suitable transcription terminator and, in eukaryotes, polyadenylation sequences operably linked to the DNA sequence encoding an alpha- glucosidase. Termination and polyadenylation sequences may suitably be derived from the same sources as the promoter.

The vector may further comprise a DNA sequence enabling the vector to replicate in the host cell. Examples of such sequences are the origins of replication of plasmids pUC19, pACYC177, pUBHO, pE194, pAMB I, and pIJ702.

The vector may also comprise a selectable marker, e.g., a gene the product of which complements a defect in the isolated host cell, such as the dal genes from B. subtilis or B. licheniformis, or a gene that confers antibiotic resistance such as, e.g. ampicillin, kanamycin, chloramphenicol or tetracycline resistance.

The procedures used to ligate the DNA construct encoding an alpha-glucosidase, the promoter, terminator and other elements, respectively, and to insert them into suitable vectors containing the information necessary for replication, are known to persons skilled in the art and readily available. See, e.g., Sambrook et al, MOLECULAR CLONING: A LABORATORY MANUAL, 2nd ed., Cold Spring Harbor, 1989, and 3^{rd} ed., 2001 ([12]).

Advantageously, the plasmid of the invention may comprise at least one other sequence encoding another protein, possibly placed in tandem with the GT-2 gene. The other coding sequence may code for any protein that may be usefully co-expressed with the GT-2 gene of the invention, for example an enzyme usually involved in the synthesis of GAGs. It may be for example at least one enzyme selected among UDP-glucose dehydrogenase KfoF and UTP-glucose-1-P-Uridyltransferase *gaLU.*

In one embodiment, the plasmid vector may comprise or consist of nucleotide sequence SEQ ID NO: 3 or SEQ ID NO : 4.
Another object of the invention relates to a host cell comprising a recombinant polynucleotide encoding Glycosyl transferase 2 from *Pyrococcus horikoshii* OT3, or its homologs from hyperthermophilic Archaea.

An isolated cell, either comprising a recombinant polynucleotide comprising sequence SEQ ID NO : 1, or an expression vector, especially a plasmid vector, as defined above, is advantageously used as a host cell in the recombinant production of a glycosyl transferase 2. The cell may be transformed with the DNA construct encoding the enzyme, for example by integrating the DNA construct, in one or more copies, in the host chromosome. Integration of the DNA constructs into the host chromosome may be performed according to conventional methods, e.g., by homologous or heterologous recombination. Alternatively, the cell may be transformed with an expression vector in connection with the different types of host cells. Introduction of a DNA construct or vector into a host cell includes techniques such as transformation; electroporation; nuclear microinjection; transduction; transfection, e.g. , lipofection mediated and DEAE-Dextrin mediated transfection; incubation with calcium phosphate DNA precipitate; high velocity bombardment with DNA-coated microprojectiles; and protoplast fusion. General transformation techniques are known in the art. See, e.g. Sambrook et al. ([12]).

Suitable host cell may be any cell known to possess the metabolic pathway necessary for the biosynthesis of precursors required for GAG production. It may be for example Archaea, Gram-positive and Gram-negative Bacteria, including *Bacillus sp., Lactococcos lactis, Agrobacterium sp., and Escherichia coli.* or even eukaryotic systems such as yeast or Chinese hamster ovary, African green monkey kidney cells, VERO cells, or the like.

Advantageously, the host cell may be *E. Coli,* for example *E. Coli* selected among ΔaraC E.coli cells, in particular LMG194 *E. Coli* strain, BL21(DE3), BL21(DE3) codon plus, BL21 (C41) and BL21 (C43) *E. Coli* strains.

Another object of the invention relates to a process for producing sulfated glycosaminoglycans (GAGs), said method comprising the steps of:
a) culturing a host cell as defined above in a culture medium containing glucose or any sugar or sugar acid synthesized from glucose, and under conditions compatible with the production of said GAGs by said cell; and
b) recovering sulfated GAGs from the culture medium of step (a).

Any host cell as defined above may be used in the process of the invention.

The medium used to cultivate the host cells may be any conventional medium suitable for growing the host cell and obtaining expression of a glycosyl transferase 2 enzyme. Suitable media and media components are available from commercial suppliers or may be prepared according to published recipes (e.g., as described in catalogues of the American Type Culture Collection).

Host cells may be cultured under suitable conditions that allow expression of a glycosyl transferase 2. Expression of the enzymes may be constitutive such that they are continually produced, or inducible, requiring a stimulus to initiate expression. In the case of inducible expression, protein production can be initiated when required by, for example, addition of an inducer substance to the culture medium, for example dexamethasone or IPTG or sophorose or using an auto-induction media which enables regulated protein expression in *E. coli* without monitoring the culture or adding inducer during cell growth (Studier, F.W. (2005) Protein Expr. Purif. 41, 207-34.2005 ([13])).

An expression host also can be cultured in the appropriate medium for the host, under aerobic or anaerobic conditions. Shaking or a combination of agitation and aeration can be provided, with production occurring at the appropriate temperature for that host, e.g. from about 25 °C to about 75°C (e.g., 30°C to 45°C), depending on the needs of the host. Culturing can occur from about 12 to about 100 hours or greater (and any hour value there between, e.g. from 24 to 72 hours). Typically, the culture broth is at a pH of about 4.0 to about 8.0, again depending on the culture conditions needed for the host relative to production of the enzyme.

For example, the process of the invention may comprises *the following steps:*
A. Culture of bacterial host cells of *Escherichia coli* LMG194 transformed with pBAD (Thermo Fisher Scientific, Invitrogen) arabinose inducible system suitable for the production in the presence of L-arabinose and/or *Escherichia coli* BL21(DE3), BL21(DE3) codon plus or BL21 (C41 or C43) strains transformed with the peT inducible system under the control of the T7 promoter suitable for the production in the presence of IPTG or auto-induction media as follows:
   a. pBAD episomal plasmid vector comprising a sequence coding for the enzyme GT2 from hyperthermophilic Archaea *Pyrococcus horikoshii* OT3 or its homologs from hyperthermophilic Archaea under the control of arabinose promoter or peT episomal plasmid vector comprising a sequence coding for the enzyme GT2 from *Pyrococcus horikoshii OT3* or its homologs from hyperthermophilic Archaea under the control of T7 promoter;
   b. pBAD episomal plasmid vector comprising a sequence coding for the enzyme GT2 from *Pyrococcus horikoshii OT3* or its homologs from hyperthermophilic archaea and a sequence coding for the enzyme UDP-glucose dehydrogenase *KfoF* in tandem under the control of the arabinose promoter or peT episomal plasmid vector comprising a sequence coding for the enzyme GT2 from *Pyrococcus horikoshii OT3* or its homologs from hyperthermophilic archaea and a sequence coding for the enzyme UDP-glucose dehydrogenase *KfoF* in tandem under the control of the T7 promoter;
   c. pBAD episomal plasmid vector comprising a sequence coding for the enzyme GT2, a sequence coding for the enzyme UDP-glucose dehydrogenase *KfoF,* a sequence coding for the enzyme UTP-glucose-1-P-Uridyltransferase *GalU* under the control of the arabinose promoter or peT episomal plasmid vector comprising a sequence coding for the enzyme GT2, a sequence coding for the enzyme UDP-glucose dehydrogenase *KfoF,* a sequence coding for the enzyme UTP-glucose-1-P-Uridyltransferase *GaLU* under the control of the T7 promoter;
B. *Escherichia coli* bacterial host cells are transformed with the plasmid vector able to produce the glycosaminoglycan and are pre-selected on ampicillin petri dishes plate
C. Recovery of Glycosaminoglycan from the culture medium.

A GAG polypeptide secreted from the host cells can be used, with minimal post-production processing, as a whole broth preparation. However, to increase the yield of GAG production, a fermentation process may be performed.

Any of the fermentation methods well known in the art can suitably used. A classical batch fermentation is a closed system, where the composition of the medium is set at the beginning of the fermentation, and the composition is not altered during the fermentation. At the beginning of the fermentation, the medium is inoculated with the desired organism(s). In other words, the entire fermentation process takes place without addition of any components to the fermentation system throughout. Alternatively, a batch fermentation qualifies as a "batch" with respect to the addition of the carbon source. Moreover, attempts are often made to control factors such as pH and oxygen concentration throughout the fermentation process. Typically the metabolite and biomass compositions of the batch system change constantly up to the time the fermentation is stopped. Within batch cultures, cells progress through a static lag phase to a high growth log phase and finally to a stationary phase, where growth rate is diminished or halted. Left untreated, cells in the stationary phase would eventually die. In general, cells in log phase are responsible for the bulk of production of product.

A suitable variation on the standard batch system is the "fed-batch fermentation" system. In this variation of a typical batch system, the substrate is added in increments as the fermentation progresses. Fed-batch systems are useful when it is known that catabolite repression would inhibit the metabolism of the cells, and/or where it is desirable to have limited amounts of substrates in the fermentation medium. Measurement of the actual substrate concentration in fed-batch systems is difficult and is therefore estimated on the basis of the changes of measurable factors, such as pH, dissolved oxygen and the partial pressure of waste gases, such as C02. Batch and fed-batch fermentations are well known in the art.

Continuous fermentation is another known method of fermentation. It is an open system where a defined fermentation medium is added continuously to a bioreactor, and an equal amount of conditioned medium is removed simultaneously for processing. Continuous fermentation generally maintains the cultures at a constant density, where cells are maintained primarily in log phase growth. Continuous fermentation allows for the modulation of one or more factors that affect cell growth and/or product concentration. For example, a limiting nutrient, such as the carbon source or nitrogen source, can be maintained at a fixed rate and all other parameters are allowed to moderate. In other systems, a number of factors affecting growth can be altered continuously while the cell concentration, measured by media turbidity, is kept constant. Continuous systems strive to maintain steady state growth conditions. Thus, cell loss due to medium being drawn off should be balanced against the cell growth rate in the fermentation. Methods of modulating nutrients and growth factors for continuous fermentation processes, as well as techniques for maximizing the rate of product formation, are well known in the art of industrial microbiology.

The produced GAG may be subjected to different purification protocols. Separation and concentration techniques are known in the art and conventional methods can be used to prepare a concentrated solution or broth comprising GAG polypeptide of the invention, such as filtration, centrifugation, microfiltration, rotary vacuum drum filtration, ultrafiltration, centrifugation followed by ultrafiltration, extraction, or chromatography, or the like, are generally used. For example, the purification method may include a SDS treatment, followed by ethanol precipitation and anionic exchange column DEAE.

Detection of submicrogram quantities of glycosaminoglycans may be performed with any method known in the art, for example on agarose gels by sequential staining with toluidine blue and Stains-All. In this example, both agarose gel electrophoresis and sequential toluidine blue/Stains-All staining can be performed to analyze all the complex glycosaminoglycans and the specificities of the glycosaminoglycan-degrading enzymes.

Following the purification, analytical chemistry technique may be performed in quality control and research for determining the content and purity of the product, as well as its molecular structure. For example, FACE analysis for determining the presence and relative abundance of individual (oligo)saccharide, nuclear magnetic resonance (NMR), FTIR (Fourier Transform Infrared Spectroscopy) analysis or mass spectrometry may be performed.

The results showed that the obtained polymers exhibited the same characteristic peak at 1.9 ppm attributed to the proton of acetyl group of either *N*-acetylglucosamine in HA or *N*-acetylgalactosamine in CS, proving that this recombinant GAG is acetylated. Biochemical characterization on agarose gel coupled to enzymatic digestion showed that this GAG polymer has the characteristics of chondroitin sulfate in terms of migration pattern, staining and enzymatic digestion. Structural determination by FTIR showed a sulfation pattern similar to chondroitin sulfate, with the presence of peaks at 860 and 1236 cm⁻¹ in the purified glycosaminoglycan corresponding to CO-S and S=O vibrations of the sulphate unit present in the chondroitin sulfate C reference and not detected in the non-sulfated hyaluronic acid reference.

Therefore, another object of the present invention is a sulfated glycosaminoglycan obtained by a process as defined above.

This sulphated GAG may be used for producing pharmaceutical, cosmetic, nutraceutical and and food products, for example as illustrated in Kubaski, F., H. Osago, R.W. Mason, S. Yamaguchi, H. Kobayashi, M. Tsuchiya, T. Orii, and S. Tomatsu, Glycosaminoglycans detection methods: Applications of mass spectrometry. Mol Genet Metab, 2017. 120(1-2): p. 67-77 ([9]), Liu, Z., F. Zhang, L. Li, G. Li, W. He, and R.J. Linhardt, Compositional analysis and structural elucidation of glycosaminoglycans in chicken eggs. Glycoconj J, 2014. 31(8): p. 593-602 ([10]) et Bi, Y., C. Hubbard, P. Purushotham, and J. Zimmer, Insights into the structure and function of membrane-integrated processive glycosyltransferases. Curr Opin Struct Biol, 2015. 34: p. 78-86 ([11]).

This invention is further illustrated by the following examples with regard to the annexed drawings that should not be construed as limiting.

### Brief description of the figures

- Figure 1 represents model of the 3D structure of PhCS-like from Pyrococcus horikoshii. The model was determined by homology with the chondroitin polymerase, (PBD ID: 2Z87). The SWISS-MODEL template library was searched with BLAST (Camacho, C., G. Coulouris, V. Avagyan, N. Ma, J. Papadopoulos, K. Bealer, and T.L. Madden, BLAST+: architecture and applications. BMC Bioinformatics, 2009. 10: p. 421 ([4])) and HHBlits (Remmert, M., A. Biegert, A. Hauser, and J. Söding, HHblits: lightning-fast iterative protein sequence searching by HMM-HMM alignment. Nat Methods, 2011. 9(2): p. 173-5 ([5])) for evolutionary related structures matching the target sequence. The Model was built based on the target-template alignment using ProMod3 (Guex, N., M.C. Peitsch, and T. Schwede, Automated comparative protein structure modeling with SWISS-MODEL and Swiss-PdbViewer: a historical perspective. Electrophoresis, 2009. 30 Suppl 1: p. S162-73 ([6])).

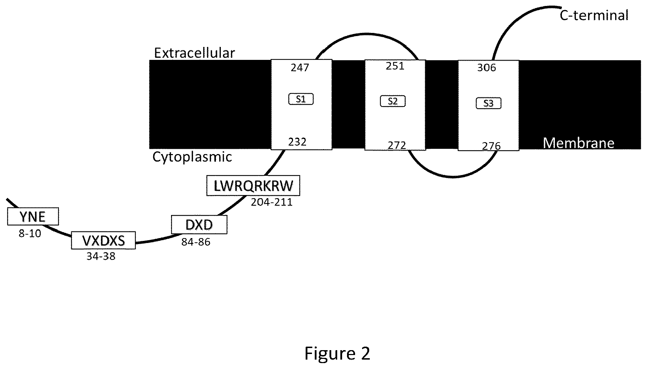

- Figure 2 represents schematic structure of PhCS-like predicted transmembrane domains. The transmembrane domains were predicted by the PSIPRED program (MEMSAT-membrane topology prediction). The catalytic motifs represented by YNE, VXDXS, DXD and LWRQRKRW were found to be located at the cytoplasmic N-terminal side.
- Figure 3: represents the pBAD-TOPO^{R} plasmid.
- Figure 4: represents the pET102/D-TOPO^{R} plasmid.
- Figure 5: represents the diagram showing the TOPO cloning sites of pBAD-TOPO^{®} vector (SEQ ID NO: 26). Restriction sites are labeled to indicate the actual cleavage site.

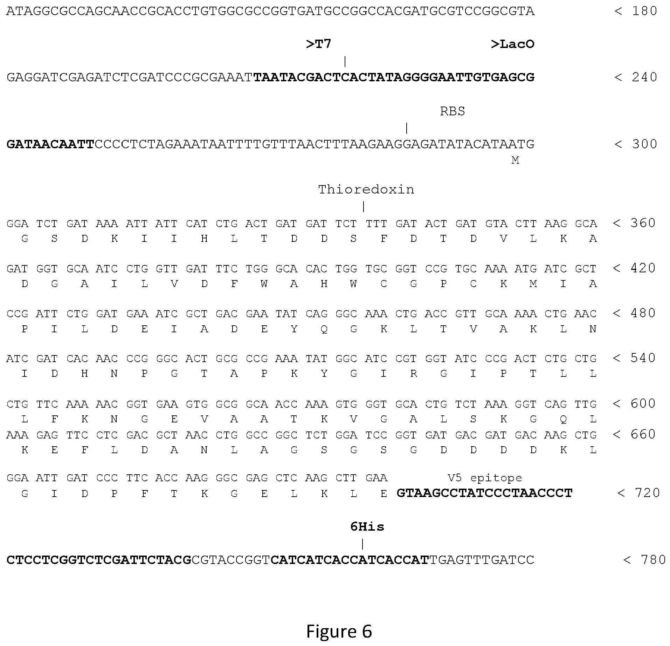

- Figure 6: represents the diagram showing the TOPO cloning sites of pET102/D-TOPO^{®} vector (SEQ ID NO: 27). Restriction sites are labeled to indicate the actual cleavage site.
- Figure 7: represents 1 % agarose gel electrophoresis showing the amplified genes. The genes Ph-CS-like (1&2) genes of length 946 bp, respectively were successfully amplified. The first well represents the ladder purchased from biotechrabbit.

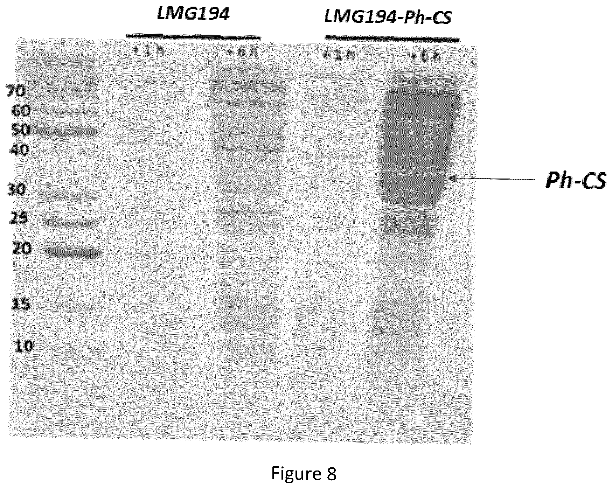

- Figure 8 represents denaturing electrophoresis gels showing results from 0.2% L-arabinose induced expression of membranous protein expressed in recombinant LMG-194 E. coli strains in a shake-flask culture. The LMG-pBAD-Ph-CS-like expresses Ph-CS-like from Pyrococcus of 37 kDa. The wild type LMG-194 represents the negative control (N.C.). The first well represents the protein ladder SDS-PAGE Standards, Broad range (in kDa.).
- Figure 9: shows estimated concentration (mg/L) of the produced GAG from the recombinant E. coli LMG-194 strains in shake flask experiments. The GAG concentration produced from LMG-pBAD-Ph-CS-like was estimated in the presence (+) or absence (-) of 0.2% L-arabinose as revealed by the CTAB turbidimetric test. The wild type LMG-194 was used as a negative control. Experiments were reproduced in duplicate.
- Figure 10 represents the estimated concentration (mg/L) of produced GAG from the two recombinant E. coli LMG-194 strains in shake flask versus bioreactor fermentation. The GAG concentration produced from LMG-pBAD-Ph-CS-like was estimated in the presence (+) or absence (-) of 0.2% L-arabinose as revealed by the CTAB turbidimetric test. Experiments were reproduced in duplicate.
- Figure 11 represents the estimated concentration (mg/L) of produced GAG in the recombinant BL21 codon plus strains in shake flask cultures. The concentration of produced GAG from pET102-Ph-CS-like was estimated in the absence and presence of IPTG as determined by the CTAB turbidimetric method. The BL21 codon plus-pET102 carrying empty vector was used as a negative control. Experiments were reproduced in triplicate.

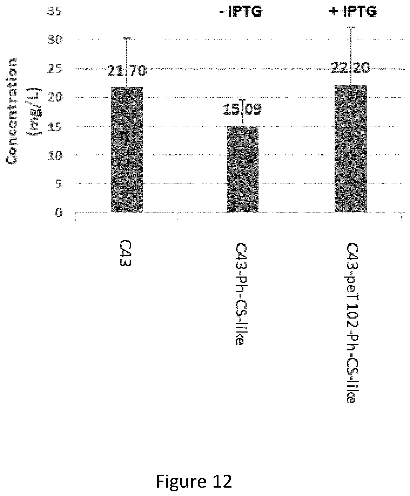

- Figure 12 represents the estimated concentration (mg/L) of produced GAG in the recombinant C43 (DE3) strains in shake flask cultures. The GAG yield produced from C43-peT102-Ph-CS-like was estimated in the absence and presence of IPTG as determined by the CTAB turbidimetric method. The C43-pET102 carrying empty vector was used as negative control. Experiments were reproduced in duplicate.
- Figure 13 represents hyaluronidase digestion of the purified polymer on agarose gel after staining with Stains-All (A) and after CTAB turbidimetric test for mass estimation of the reaction product (mg) formed after HAase digestion from the DEAE purified fractions from LMG-pBAD-Ph-CS-like (B). Experiments were reproduced in duplicate.

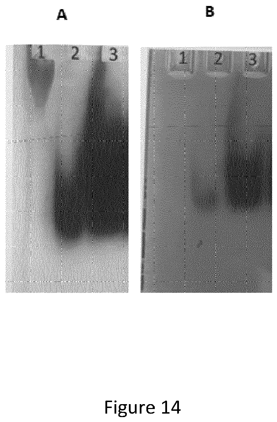

- Figure 14: Agarose gel electrophoresis of standard commercial GAGs and the heterologous biopolymers stained with (B)toluidine blue. Commercial GAGs (1: hyaluronic acid; 2: chondroitin 6-sulfate) and the heterologous biopolymers (3: combined fractions 14 and 15 from E. coli LMG-PhCS-like.
- Figure 15 represents FTIR characterization of purified biopolymer produced by recombinant E. coli strains represented by LMG-pBAD-PhCS-like.
- Figure 16 represents FTIR characterization of chondroitin 6-sulfate purchased from Carbosynth.
- Figure 17 represents One-dimensional NMR spectral analysis of (a) chondroitin 6-sulfate (CS) and (b) hyaluronic acid (HA) standards.
- Figure 18 one-dimensional NMR spectral analysis of fractions contaninig the polymer of interest after SDS treatment, ethanol precipitation and anion exchange chromatography. Fractions were eluted between 0.35 and 0.45 M NaCl from the recombinant strains LMG-pBAD-PhCS-like.
- Figure 19 represents mass spectroscopy using ESI ionization (negative ion mode) to identify GAG oligosaccharides. Chondroitin sulphate oligosaccharides and disaccharides were obtained after hyaluronidase digestion. The products were compared with the standard of chondroitin-6-sulphated products. (a) CS-C, (b) polymer from LMG-pBAD-PhCS-like and (1) and after (2) digestion with hyaluronidase.

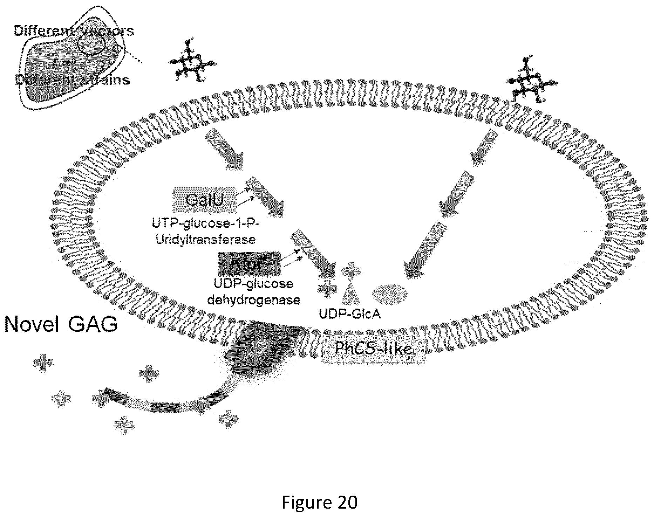

- Figure 20 represents the general strategy for metabolic engineering of *E.coli* for the production of chondroitin sulfate.

### Examples

### Example 1 : Genetic engineering of E. coli for the production of a novel glycosaminoglycan

### 1. DNA manipulations, bacterial strains and growth conditions

### 1.1. Bacterial strains and growth conditions

### 1.1.1. Media

**Luria Bertani Broth, Lennox** (Conda, Cat No. 1231.00), composition: 10 g.L⁻¹ tryptone, 5 g.L⁻¹ yeast extract and 5 g.L⁻¹ NaCl. The pH of this solution was adjusted to 7 to 25°C.

**Luria Bertani Broth, Miller** (HIMEDIA, Cat No. M1245-500G), composition: 10 g.L⁻¹ tryptone, 5 g.L⁻¹ yeast extract and 10 g.L⁻¹ NaCl. The pH of this solution was adjusted to 7.5 at 25°C. This medium is characterized by the presence of a double amount of NaCl with respect to LB Lennox. Furthermore, addition of agar (15 g.L⁻¹) to LB creates a gel for bacteria to grow upon, and is therefore used for plating bacterial cultures on petri dishes.

**Hanahan's Broth or Super Optimal Broth Medium or SOB medium** (HIMEDIA, Cat.No. M1252-500G), composition: 20 g.L⁻¹ tryptone, 5 g.L⁻¹ yeast extract, 0.5 g.L⁻¹ NaCl, 2.4 g.L⁻¹ MgSO₄ and 0.186 g.L⁻¹ KCI. The pH of this solution was adjusted to 7 at 25°C.

**Terrific broth (TB),** composition: 12 g.L⁻¹ tryptone, 24 g.L⁻¹ yeast extract and 4 mL/L glycerol. After autoclave, a filtered sterile solution of 0.072 M K₂HPO₄.3H₂O and 0.017 M KH₂PO₄ was added to the medium.

All culture media were autoclaved for 15 min at 121°C and cooled. The appropriate antibiotic was added at the desired concentration.

### 1.1.2. Antibiotics

All prepared antibiotic solutions were sterile filtered through a syringe filter (0.22 µm and 25 mm diameter) (StarLab Scientific, Cat.No. SLCA2522S) and stored at -20°C before use.

**Ampicillin (Sigma-Aldrich, Cat No.10835242001):** The stock solution was prepared at 50 mg/mL in water and used at a final concentration of 100 µg/mL.

**Chloramphenicol (Sigma-Aldrich, Cat No. C0378):** The stock solution was prepared at a concentration of 50 mg/mL in ethanol and used at a final concentration of 35 µg/mL.

**Kanamycin (Sigma-Aldrich, Cat No. 60615):** The stock solution was prepared at a concentration of 100 mg/mL in water and used at a final concentration of 100 µg /mL.

**Doxycycline (Sigma-Aldrich, Cat No. D1822):** The stock solution was prepared at a concentration of 20 mg/mL in water and used at a final concentration of 10 µg/mL.

### 1.1.3. E. coli strains

**DH5-α strain (Invitrogen, Cat No. 18265017):** This cloning strain is developed by D. Hanahan with multiple mutations that enable high-efficiency transformations. The genotype is F-endA1 glnV44 thi-1 recA1 relA1 gyrA96 deoR nupG purB20 ϕ80dlacZΔM15 Δ (lacZYA-argF) U169, hsdR17 (rK-mK +) / λ- [44].

**TOP10 strain (Invitrogen, Cat No. C404003):** This strain is used also for general cloning of blunt-end PCR products into the pBADTOPO^{®} vector. The genotype is: F- mcrA Δ(mrr-hsdRMS-mcrBC) φ80lacZΔM15 ΔlacX74 recA1 araD139 Δ(ara-leu)7697 galU galK rpsL (StrR) endA1 nupG. **LMG-194 strain (Invitrogen, Cat No. 1923908):** They are ara mutant strains with genotype [F- ΔlacX74 galE thi rpsL ΔphoA (Pvu II) Δara714 leu::Tn10]. They are expression hosts used in combination with vectors containing pBAD promoter. These strains are also streptomycin and tetracycline resistant.

**BL21 codon plus strain (DE3) RIPL (Stratagene, Cat No.230280):** This strain, derivative of *E*. *coli* B, is a general protein expression strain that lack both the Lon protease and the OmpT protease. The genotype of *E*. *coli* B [F- ompT hsdS(rB - mB -) dcm+ Tetr gal λ(DE3) endA Hte [argU proL Camr ] [argU ileY leuW Strep/Specr]. It harbors a prophage DE3 derived from a bacteriophage λ, which carries the T7 RNA polymerase gene under the control of the **lac**UV5 promoter. The cells also harbor extra copies of the argU, ileY, and leuW as well as the proL tRNA genes.

**C43 (DE3) (Lucigen, Cat No. 60446-1):** This strain is derived from the T7 RNA polymerase (P)-based BL21(DE3) protein production strain. It is a mutant strain of BL21 (DE3) and carries mutations that lower T7 RNAP expression levels that result in strongly reduced T7 RNAP accumulation levels.

### 1.2 Gene sequences

The gene sequence of Ph-CS-like, Pyrococcus horikoshii OT3 is as follow:
Ph-CS-like, Pyrococcus horikoshii OT3, Accession number PH_RS02140 SEQ ID NO: 1 :

GTGAGCATAATAATCCCAGCTTATAATGAAGAGGAGAACATAAAAAGA
GCAATTAAAGCTGCTCTTTCTCAGGATTATCCAGTTAGCGAGGTAATA
GTTGTGGACGATGGGAGCGAGGATGGAACGTACGAGAGGGCCAAGG
AAATTAAAGATCATAGACTTAAGGTACTGAAGATAGATCATAGGGGAA
AAGCGGGGGCGATAAATGAGGGTCTTAAAATCGCCAAGGAAGTGTG
ATCGTAACTACCGATGCGGACTCTTTCATGAGCAAGGATGCAGTTAG
GAGGTTAGTTGAGAGGTTTTACTCCGATGATGTTGTTGCAGTTGGGG
GCCAAATAAGGGTCATCGTTGAATCCTTCATGACGCTCGTTCAGGATA

TAGAGCACCTGAGAATTGCGATGTATAGGAGAGGGAAGGAGCTTGAA
AATTTAAGCTTAGCCCCGGTCCCCTTTCCGCTTTCAGAAGGATGTT
CTCGAAAAGATCGGTGGGATACAGCGTAGTATAGTTGAAGATTACGC
CACGACTAAGATTGTAAAGAAGTATGGAAGGGTTGTTTACGCGCCGA
AGGCTAAACTCTACACTAGAATGCCTATCACCCTTCGAGATCTATGGA
GGCAACGAAAGAGATGGTTCTTGGGGGATCTTCAGCACCATAACCTG
AATGACCTTCTGAATCTACTCCTTGGGGATGTAATAGCCCTCCTAGAT
GTGATACTTCCCTTCATCTTCCTATTAGAGGGTAGTTATCTCCCCCTTT
TAGCCTTCGTGATGTTCGAAGTAATAACCATGGTGATAGCGGTAATCT
GGGAGGGAGGCTCCTTAATCGAGGCCCTGCTTTTCCCCGTTGTCCTC
TGGTTCTTGGCCCTCTTCTACCTTTCCCTCCACTTATACGGATACGCT
GTCTATGGCTTGAAAAGTTTGCTTAGATTCACTCGTTCCTAG

### 1.3 Plasmid vectors

Two plasmids were used in this study for the production of recombinant GAG

### 1.3.1 pBAD system

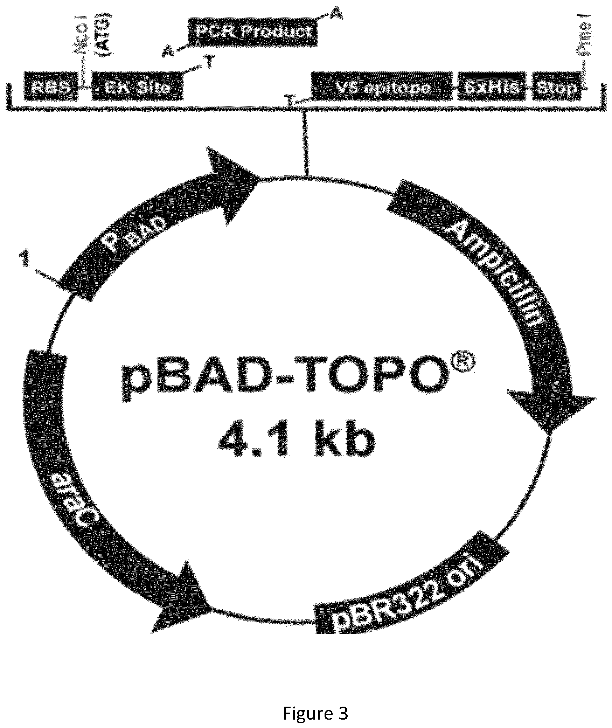

The pBAD-TOPO^{®} (Invitrogen, Catalog No. K4300-01) is a vector of 4,126 base pairs. It is shown in Figure 3. The pBAD plasmids harbors *Ph-CS-like* genes from *Pyrococcus horikoshii.* The corresponding gene sequence of Ph-CS-like, *Pyrococcus horikoshii* OT3 (Accession number PH_RS02140) is SEQ ID NO: 1. The PCR product was amplified with a stop codon within the open reading frame which provides the expression of the gene without the 6x-Histidine tag. It was designated LMG-pBAD-PhCS-like.

There is no additional Tags at the *N*-terminal and C-terminal. The selection marker is Ampicillin. The promoter is a weak promoter (araBAD). Induction is possible by L-arabinose.

Figure 3 shows the TOPO cloning sites of pBAD-TOPO^{®} vector. Restriction sites are labeled to indicate the actual cleavage site.

### 1.3.2 pET system

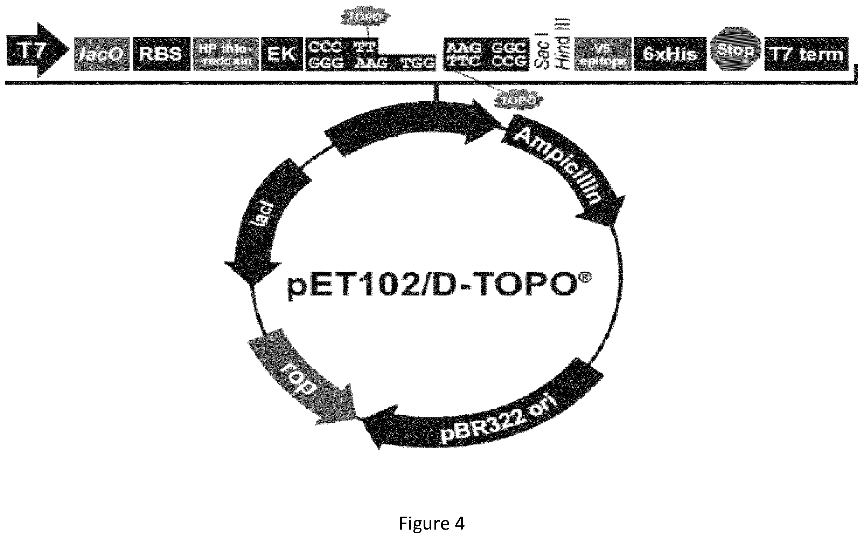

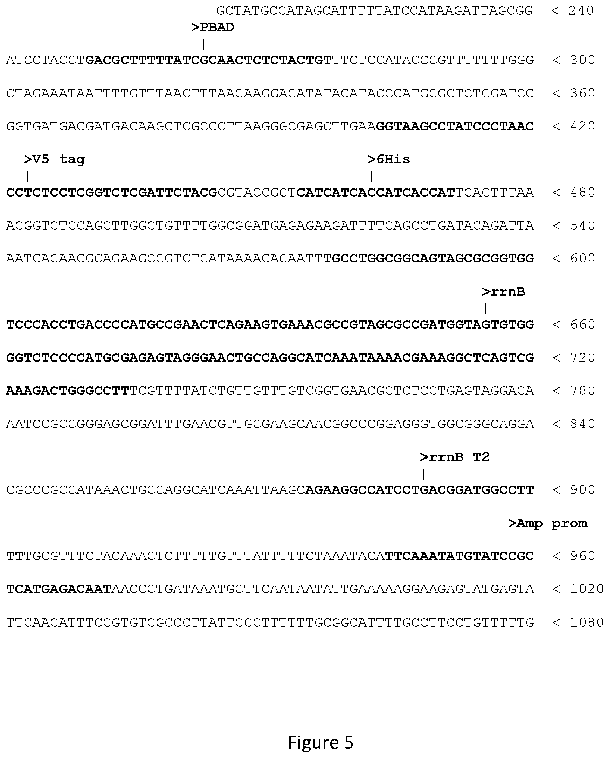

The pET102/D-TOPO^{®} vector (Invitrogen, Catalog No. K102-01) is based on the strong T7 promoter, with IPTG (isopropyl-1-thio-β-D-galactoside) as the inducible agent. Target protein expression may be initiated by transferring the plasmid into an expression host containing a chromosomal copy of the T7 RNA polymerase gene under lacUV5 control (DE3 strain). The expression is induced by the addition of IPTG to the bacterial culture. The gene of interest was cloned as a fusion to the only *N*-terminal His-Patch thioredoxin for increased translation efficiency and solubility of heterologous proteins. Selection marker is Ampicillin. Its structure is shown in Figure 4. The diagram of Figure 5 shows the TOPO cloning sites of pET102/D-TOPO^{®} vector. Restriction sites are labeled to indicate the actual cleavage site.

### 1.4 Preparation of competent Escherichia coli cells

The CCMB80 buffer was prepared by adding 0.98 g KOAc (10 mM), 11.8 g CaCl₂.2H₂O (80 mM), 4.0 g MnCl₂.4H₂O (20 mM), 2.0 g MgCl₂.6H₂O (10 mM), 100 g glycerol (10%), and water to a final volume of 1 L. The pH of solution was adjusted to 6.4 then sterile filtered and stored at 4°C. A single colony of DH5-α or TOP10 (Invitrogen) was inoculated into 5 mL of SOB medium and incubated overnight at 37°C with vigorous shaking at 200-220 rpm. The preculture was diluted by 1/100 to reach an optical density of OD₆₀₀ of 0.3. The cells were then centrifuged at 3000 rpm and 4°C for 10 min. The supernatant was discarded and the cells were resuspended in 10 mL of ice cold CCMB80 buffer with a gentle swirl. Then the resuspended cells were incubated on ice for 20 min, centrifuged as before then resuspended in 5 mL of ice cold CCMB80 buffer. Each 100 µL was aliquoted into microcentrifuge tube and freezed at -80°C.

### 1.5 Transformation of competent cells

100 µL of chemically competent cells were incubated with 1 µL of plasmid DNA on ice for 30 min. Heat shock was performed at 42°C for 1 min 30 s and then returned to ice immediately for 5 min. 400 µL of SOB medium was then added to the cells to be incubated in the shaker for 1 h at 37°C with agitation of 220 rpm. After incubation, transformed cells were plated on LB agar plates supplemented with the appropriate antibiotic.

### 2. Cloning reactions

### 2.1 Cloning of Ph-CS-like in pBAD vector

For cloning of Pyr-CS-like along with the pBAD plasmid, a series of forward and reverse primers were designed specifically for each DNA of interest, as listed in Table 2.

**Table 1: Primers used in this study to amplify the genes of interest**

| ***Primer name*** | ***Primer sequence (5'*→*3')*** |
|---|---|
| Ph-CS-like-FW | GTGAGCATAATAATCCCAGC (SEQ ID NO : 5) |
| Ph-CS-like-Re | CTAGGAACGAGTGAATCTAAGC (SEQ ID NO : 6) |
| pBAD Forward | ATGCCATAGCATTTTTATCC (SEQ ID NO : 7) |
| pBAD Reverse | GATTTAATCTGTATCAGG (SEQ ID NO : 8) |

The DNA fragment coding for chondroitin synthase-like from *Pyrococcus horikoshii* was amplified by PCR using the forward and reverse primers listed in table 2. The PCR was carried out using a mixture called "RedTaq Ready Mix" (purchased from Sigma) that contains the Taq DNA polymerase enzyme, the dNTPs and a buffer formed of Mg⁺⁺ and NaCl. Each reaction was carried out in a total volume of 40 µl with 20 µl of RedTaq Ready Mix, 1 µl primer forward, 1 µl primer reverse, 17.5 µl sterile water. The CS-like gene amplification program was as follows: initial denaturation at 95 ° C for 1 min, followed by 30 cycles of denaturation at 95 ° C for 1 min, hybridization at 58 ° C for 30 seconds and extension at 72 ° C for 1 min, followed by a final extension for 7 min at 72 ° C needed for the addition of a single deoxyadenosine at the 3 'end of the PCR product required for the TOPO cloning and to ensure the complete extension of the amplified fragments. After amplification, the PCR products were migrated on 1% agarose by agarose gel electrophoresis to confirm the expected size of the desired DNA fragment.

### 2.2 Perform the TOPO Cloning Reaction and transformation TOP10 chemically competent E. coli

The TOPO Cloning reaction was performed by adding 2 µl of fresh PCR product, 1 µl of vector pBAD, 1 µl of salt solution which allow to increase the transformation efficiency and 2 µl of sterile water, then incubated for 15 min at room temperature and placed on ice to proceed in the transformation of One Shot TOP10 chemically competent *E*. *coli.* 3µl of the TOPO cloning reaction was added into a vial of One Shot TOP10 chemically competent *E*. *coli* and mixed gently, incubated on ice for 30 minutes then the cells were heat shocked for 1min 30 s at 42°C and transferred to ice immediately. Then SOB medium was added to the cells and grown at 37°C for 1 hour, then 200 µl of bacterial culture was plated on LB agar plates supplemented with ampicillin (50 µg/ml) and incubated overnight at 35°C. After purification of DNA plasmid by GenElute HP Plasmid Miniprep, the final sequence was confirmed by PCR with primers of P-seq-F as forward and HAS-PHO Re as reverse primerThe use of a combination of the pBAD Forward and pBAD Reverse sequencing primers and a primer that hybridizes within the insert is important to confirm that the gene is cloned in the correct orientation. The pBAD Forward and pBAD Reverse sequencing primers are listed in the Table 2. After verification of positive clones, the competent *E*. *coli* LMG 194 strains were transformed with the vectors containing the desired plasmid LMG-pBAD-Ph-CS-like and selected on LB agar plates supplemented with ampicillin (100 µg/ml). The plasmids were sent for sequencing to verify the presence of the correct sequence insert.

### 2.3 Cloning of an operon containing chondroitin synthase with KfoF and galU genes

### PCR amplification of KfoF and galU

The genome of *E*. *coli* LMG-194 was used as a template for the amplification of *KfoF* and *galU* genes by PCR (PCR1) using specific primers as shown in Table 3. These primers were deisgned to recognize *KfoF (UDP-glucose dehydrogenase NCBI-GeneID:946571)* and galU *(UDP-glucose-phosphorylase NCBI-GeneID:945730)* and that added in addition a ribosome binding site (RBS) to increase internal translational efficiency (Table 3). All the primers to be used with the HiFi DNA assembly kit (BioLabs, Cat No. E5520S) were generated through the program Nebbuilder available online www.nebuilder.neb.com. Serial cloner was also used to check for the obtained reading frames from the final construct of the plasmid containing the operons (Serial cloner, version 2.6; http://serialbasics.free.fr/Serial Cloner.html). After the first PCR, a second PCR (PCR2) was applied on the PCR1 products to add specific ends that overlap to the vector or to the adjacent gene. PCR2 added to the *KfoF* gene one overlapping end with the plasmid pBAD and another overlapping end either with the plasmid (to have a *KfoF-galU* operon) or with the GalU gene (to have a KfoF-galU operon). For the *galU* gene, PCR2 added one overlapping end with the *KfoF* gene and another overlapping end with the pBAD plasmid. These overlapping sequences are necessary for the cloning reactions of the two genes in the plasmid pBAD. The PCR reactions were performed using a mixture called "RedTaq Ready Mix" (purchased from Sigma) containing the *Taq* DNA polymerase enzyme, the dNTPs and a buffer of Mg²⁺ and NaCl. The PCR conditions are summarized in the Table 3.

**Table 3: Conditions of PCR1 and PCR2 for KFOF and GALU genes.**

| | **PCR conditions** | **PCR1 *KFOFIGALU*** | **PCR2 *KFOFIGALU*** |
|---|---|---|---|
| | Initial denaturation | 95°C, 2 min | 95°C, 2 min |
| 45 cycles | Denaturation | 95°C, 1 min | 95°C, 1 min |
| | Hybrid ization | 50°C, 1 min | 55°C, 1 min |
| | Extension | 72°C, 1 min | 72°C, 2 min |
| | Final extension | 4°C ∞ | 4°C ∞ |

Table 4 shows the primers design for PCR1 and PCR2 of KFOF and GALU genes.

**Table 4:**

| | **Gene** | **Primers** | **Primer sequence (5'→3')** |
|---|---|---|---|
| PCR1 | *KFOF* | KFOF-GEN-RBS-FW | GGAGATATACATACCCATGAA AATCACCATTTCCG (SEQ ID NO : 9) |
| | | KFOF-GEN-REV-STOP | TTAGTCGCTGCCAAAGAGAT C (SEQ ID NO : 10) |
| | *GALU* | GALU-GEN-RBS-FW | GGAGATATACATACCCATGG CTGCCATTAATACG (SEQ ID NO : 11) |
| | | GALU-GEN-REV-STOP | TTACTTCTTAATGCCCATCTC TTC (SEQ ID NO : 12) |
| PCR2 | *KFOF* | PBAD-TOPO/KFOF-FWD | TCACCATCACCATTGAGTTTT TAAGAAGGAGATATACATACC C (SEQ ID NO : 13) |
| | | KFOF/PBAD-TOPO-RE | AGCCAAGCTGGAGACCGTTT TTAGTCGCTGCCAAAGAG (SEQ ID NO : 14) |
| | | KFOF/GALU-KFOF-RE | CCTTCTTAAATTAGTCGCTGC CAAAGAG (SEQ ID NO : 15) |
| | *GALU* | KFOF/GALU-GALU-FWD | 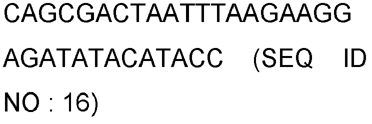 |
| | | PBAD-TOPO/GALU-RE | 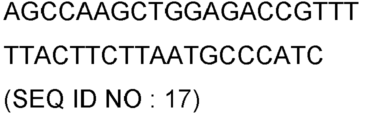 |

Each reaction was carried out in a total volume of 40 µL with 20 µL of RedTaq Ready Mix, 1 µL forward primer, 1 µL reverse primer, 1 µL matrix DNA and 17.5 µL sterile water. After amplification, the PCR1 and PCR2 products were migrated on 1% agarose gel electrophoresis at 100 V for 1 h to confirm the presence of desired DNA fragment with the correct length using the 1 kb DNA ladder (NIPPON Genetics Europe, Cat No. MWD1P).

### 2.3.1 Cloning of KfoF and gaLU genes into pBAD vector

### 2.3.1.1 DNA cloning by PCR

DNA cloning method was achieved by PCR. The necessary primers needed for the amplification and linearization of vectors harboring *Ph-CS-like* gene from *Pyrococcus* and the primers required to add specific ends to *KFOF* gene to overlap with *PhCS-like* genes from one side and with the pBAD vector from the other side were designed. These overlapping sequences are useful for the cloning reactions of the two genes in the plasmid pBAD. The primers used for the amplification of vectors as well as the *KFOF* gene (3 and 4) are listed in Table 5.

**Table 5: Primers used for cloning by PCR strategy .**

| | **Primer name** | **Primer sequence (5'→3')** |
|---|---|---|
| 1 | pBAD-linear_fwd | AACGGTCTCCAGCTTGGC (SEQ ID NO: 18) |
| 2 | pBAD-linear_rev | TAAACTCAATGGTGATGG (SEQ ID NO: 19) |
| 3 | KFOF_overlappBAD-Fwd | caccatcaccattgagtttaTAAGAAGGAGATATACA TACCC (SEQ ID NO: 20) |
| 4 | KFOF-overlappBAD-Re | cagccaagctggagaccgttTTAGTCGCTGCCAAAG AG (SEQ ID NO: 21) |

The Long Range PCR (Thermo SCIENTIFIC, Cat No. MAN0016323) allows the amplification of DNA lengths up to 15 kb or longer genomic DNA. Each reaction was carried out in a total volume of 50 µL with 5 µL of dNTP mix, 1 µL of Long PCR Enzyme Mix, 5 µL of 10X Long PCR buffer with 15 mM MgCl₂, 2 µL of DMSO, 1 µL forward primer, 1 µL reverse primer, 0.5 µL of template DNA and 34.5 µL of sterile water. The amplification program consists of three steps cycling protocol listed in the Table 6.

**Table 62 : Three-step cycling protocol.**

| **Step** | **Temperature °C** | **Time** | **Number of cycles** |
|---|---|---|---|
| Initial denaturation | 94 | 1min | 1 |
| Denaturation | 94 | 20 s | 10 |
| Annealing | 57 | 30 s | |
| Extension | 68 | 6 min | |
| Denaturation | 94 | 20 s | 20 |
| Annealing | 57 | 30 s | |
| Extension | 68 | 6 min | |
| Final extension | 68 | 10 min | 1 |

After amplification, the PCR products were migrated on 1% agarose by agarose gel electrophoresis for 1 h at 100 V to confirm the expected size of the desired DNA fragment. Then, the linearized plasmids were digested with *DpnI* enzyme (Thermo SCIENTIFIC, Cat No. ER1701) that cleaves the parental template Dam methylase-methylated plasmid DNA from E. *coli.* In a 10 µL reaction, 5 µL of PCR product was mixed with 1 µL of 10X buffer Tango (Thermo SCIENTIFIC, Cat No. BY5) and 1 µL *DpnI* enzyme with 3 µL of sterile water. Each mix was incubated for 1 h at 37°C and heat-inactivated by incubating at 80°C for 20 min.

### 2.3.1.2 Assembly of the linearized plasmid and the KfoF and galU genes by the Hifi DNA assembly kit

The linearized plasmids (obtained by PCR or restriction enzymes) were purified by gel extraction kit (Sigma-Aldrich, Cat No. NA1111). After purification, the *KFOF* and *GALU* genes were cloned into the linear pBAD plasmid according to the manufacturer's instructions using the NEBuilder HiFi DNA Assembly protocol (BioLabs, Cat No. E5520S) to obtain synthetic operons CS-like-KfoF and CS-like-KfoF-GalU for each strain. Then, the competent DH5-α strains were transformed by thermal shock with each reaction of assembly. The pUC vector was included as a positive control to verify the transformation efficiency of competent cells. The transformed DH5-α bacteria were incubated at 37°C for 40 min, then inoculated on TB agar plates supplemented with ampicillin 100 µg/mL and incubated overnight at 35°C.

### 2.3.1.3 Purification of the plasmid containing the synthetic operon and PCR analysis

After assembly and cloning reactions, the plasmids were purified using GenElute HP Plasmid Miniprep purchased from Sigma-Aldrich, and the presence of *KFOF* and *GALU* genes was analyzed by PCR using specific primers for each gene (Table 7). The PCR conditions are illustrated in Table 8.

**Table 7: Primers used in this study for amplifying KFOF and GALU genes.**

| **Plasmid** | **Primer name** | **Primer sequence (5'→3')** |
|---|---|---|
| | pBAD-TOPO/KFOF-Fw | |
| pBAD-Ph-CS-like-Pyr-KFOF | | TCACCATCACCATTGAGT TTTTAAGAAGGAGATATA CATACCC (SEQ ID NO: 22) |
| | KFOF/pBAD-TOPO-RE | AGCCAAGCTGGAGACCG TTTTTAGTCGCTGCCAAA GAG (SEQ ID NO: 23) |

**Table 8 : PCR conditions for the amplification of KFOF and GALU genes**

| **Step** | **Temperature °C** | **Time** | **Number of cycles** |
|---|---|---|---|
| Denaturation | 95 | 2 min | 45 |
| Annealing | 55 | 1 min | |
| Extension | 94 | 1min | |
| Final extension | 72 | 2 min | 1 |

### 2.4 Cloning into pET vector

### 2.4.1 Amplification of Ph-CS-like genes

The first step of cloning reaction consists of amplifying the target genes. The DNA fragment coding for *Ph-CS-like* genes from *Pyrococcus* was amplified by PCR, using the forward primer containing on the 5' end the 4 base pair sequences (CACC), necessary for directional cloning of the forward primer, in a way such our gene of interest will be optimally expressed and fused in frame with the vector (Table 9).

**Table 9: Primers used in this study to amplify gene of interest**

| **Primer name** | **Primer sequence (5'→3')** |
|---|---|
| Ph-CS-like-pET102-TOPO-direct-Fwd | **CACC**GTGAGCATAATAATCCCAGC (SEQ ID NO: 24) |
| Ph-CS-like-pET102-TOPO-direct-Re | CTAGGAACGAGTGAATCTAAGC (SEQ ID NO: 25) |

PCR was carried out with 50 µL of reaction mixture containing 1 µL template DNA, 2.5 µL each primer (10 mM), 19 µL of sterile water, and 25 µL *Taq* DNA polymerase (Sigma-Aldrich). The PCR assay was performed for 40 cycles under the following conditions, See Table 10.

**Table 10: PCR conditions for the amplification of Ph-CS-like gene in pET102 vector.**

| **Step** | **Temperature °C** | **Time** | **Number of cycles** |
|---|---|---|---|
| Initial denaturation | 95 | 5 min | 1 |
| Denaturation | 95 | 1 min | 40 |
| Annealing | 58 (*Ph-CS-like*) | 1 min 30 s | |
| Extension | 72 | 2 min | |
| Final extension | 72 | 7 min | 1 |

### 2.4.2 Cloning reaction and transformation of chemically competent E. coli with pET102-TOPO vector

The TOPO cloning reaction was performed by adding 4 µL of fresh PCR product, 1 µL of vector pET102/D-TOPO^{®} and 1 µL of salt solution, then incubated for 15 min at room temperature and placed on ice to proceed in the transformation of One Shot TOP10 chemically competent *E. coli.* 3 µL of the TOPO cloning reaction was added into a vial of One Shot TOP10 chemically competent *E. coli* and mixed gently, then incubated on ice for 30 min. The cells were then heat shocked for 1 min 30 s at 42°C and transferred to ice immediately. Then SOB medium was added to the cells and grown at 37°C for 1 h, then 200 µL of bacterial culture was plated on LB agar plates with ampicillin (100 µg/mL) and incubated overnight at 35°C. Several colonies were then isolated, and their plasmids DNA were purified by Miniprep (Sigma-Aldrich, Cat No. NA0150) and PCR was performed to verify the presence of the insert. These plasmids harboring *Ph-CS-like* gene were then transformed in either BL21 codon plus or C43 (DE3) competent *E. coli* strains and selected on LB agar plates with ampicillin (100 µg/mL) and chloramphenicol (35 µg/mL).

### II. Expression and characterization of the recombinant proteins in E. coli

### 1. Expression and characterization of recombinant PhCS-like protein from Pyrococcus horikoshii

### 1.1. Production of recombinant proteins in E. coli

In order to follow the expression of *PhCS-like* recombinant protein expressed in LMG-194, BL21 codon plus and C43 (DE3) *E. coli* expression strains on SDS-PAGE, bacteria transformed with the appropriate vector harboring *Ph-CS-like* genes from *Pyrococccus* were grown overnight in shake flask experiments at 37°C with shaking at 235 rpm in SOB medium supplemented with the appropriate antibiotics. Cultures were then diluted by 100 and grown to reach an optical density (OD₆₀₀) of 0.6 upon which the cultures were splitted a part (Non-induced and induced) and gene expression was induced using either 0.2% L-arabinose for LMG-194 or 1 mM IPTG for BL21 codon plus and C43 (DE3), and the cultures were left to grow for additional 24 h.

### 1.2. Detection of PhCS-like protein expression on SDS-PAGE

The polyacrylamide gel electrophoresis (PAGE) provides a versatile and high resolution method for fractionation of proteins on the basis of size. Each component migrates as a band with characteristic electrophoretic migration rate depending on its size. The use of sodium dodecyl sulfate (SDS), a detergent with a strong protein-denaturing effect, and polyacrylamide gel largely eliminates the influence of the structure and charge, and proteins are separated solely based on polypeptide chain length. The SDS denatures secondary and nondisulfide-linked tertiary structures and coats them with a negative charge that correlates with their length, allowing molecular weights to be estimated. The staining of the gel subsequent to electrophoresis reveals the characteristic band positions.

Aliquots (1 mL) of the non-induced and induced strains were taken after 3, 6 and 24 h of induction, centrifuged and the pellet was resuspended in an equal volume of laemmli buffer composed of 4% SDS, 20% glycerol, 10% 2-mercaptoethanol, 0.004% bromphenol blue and 0.125 M Tris HCl of pH 6.8 (Sigma-Aldrich, Cat No. SLBX2653), heated for 5 min at 95°C and loaded on the gel then migrated at 100 V for 30 min and 130 V for 1 h in running buffer composed of 25 mM tris-base, 192 mM glycine and 0.1 % SDS. The concentration of the acrylamide gel depends on the protein size. The stacking gel is the upper part for loading the sample. Proteins are well lined up when they reach the lower gel (Resolving gel). The stacking gel (6 %) was prepared in total volume of 5 mL by mixing 30 % acrylamide solution, 0.126 M Tris-CI pH 6.8, 0.0035 M SDS, 0.00219 M APS, 0.0144 TEMED and H₂O. The resolving gel (12.5 %) was prepared in a total volume of 7.5 mL by mixing 30 % acrylamide solution, 0.4 M Tris-CI pH 8.8, 0.003 M SDS, 0.002 M APS, 0.0096 M TEMED (N,N,N',N'-Tetramethylethylenediamine) and H₂O. Once the electrophoresis is complete, the gel was stained with Brilliant Blue R Staining (0.25 g Coomassie Brilliant Blue R (Sigma-Aldrich, Cat No. 1.12553) in 45% methanol and 10% acetic acid) for 20 min with slight agitation at room temperature to make the separated proteins appear as distinct colored bands on the gel. Finally, the gel was destained with 10% acetic acid and 40% ethanol. The size of the protein was estimated in comparison with the molecular weight marker bands (Thermoscientific, Cat No. 26619).

### 2.1. Cetyltrimethylammonium bromide turbidimetric method (CTM assay)

In oder to determine the enzymatic activity on the total protein extracts of both recombinant *E. coli* strains, CTM assay was performed to estimate the concentration of the produced reaction product. This test is based on the formation of turbidity between the negatively charged polyanionic polysaccharide reaction product resulting from the polymerizing activity of the recombinant enzyme, and cetyltrimethylammonium bromide (CTAB), which is a cation surface active agent. Therefore, the turbidity resulting from the precipitation is related to the concentration of reaction products and can be titrated by spectrophotometry and the concentration of the formed reaction products (in this case, resulting from the polymerizing activity of the recombinant enzyme) present in each reaction was calculated based on a calibration line of HA.

The CTAB reagent (2.5 g) was dissolved in 100 mL of 2% (w/v) NaOH. Different concentrations of HA standard solutions ranging from 0 to 1 mg/mL were prepared in order to check the linearity range of the standard curve. 250 µL of each concentration was introduced into a cuvette containing 500 µL of CTM reagent and was incubated at 37°C for 10 min. Same conditions were applied to the samples. Absorbance was read at 600 nm against the blank (HA solution replaced by 0.05 M NaCl solution) and plotted against HA standard concentrations to constitute calibration lines.

### 2.2. Agarose gel electrophoresis

We intended to follow the formation of polymer resulting from the polymerizing activity of both recombinant proteins expressed in the various *E. coli* strains on agarose gel electrophoresis followed by subsequent staining with Stains-all that is usually used to stain and identify the non-sulfated hyaluronic acid and other GAGs with basis on their specific colors. Following enzymatic activity, each sample was lyophilized and resuspended in loading dye (LD) composed of 0.2% bromophenol blue in 10 M formamide. Around 5 µg of each sample was loaded on 1% agarose gel that was prepared in a 0.5 cm thickness apparatus of a vertical protein gel polyarcrylamide electrophoresis and was pre-run for 2 h at 40 V in TAE buffer composed of 40 mM Tris base, 20 mM acetic acid, 1 mM EDTA disodium salt in 1 L of deionized water. After samples loading, the gel was run for 30 min at 20 V then at 40 V for 2-3 h. Immediatly after the run, the gel was stained with stains-all staining solution Stains-all dye that is usually used to stain and identify the non-sulfated hyaluronic acid and other GAGs with basis on their specific colors. This stain is composed of 0.0055% stains-All in 50% ethanol and incubated in the dark ON at room temperature to be then destained with 10% ethanol for 1 h to detect the presence of the reaction biopolymer product.

### III. Production and characterization of the glycosaminoglycan polymer from recombinant E. coli and characterization of the produced glycosaminoglycan

### 1. Production of glycosaminoglycan in recombinant E. coli strains

### 1.1. GAG production in LMG-194 recombinant strain

### 1.1.1. Production of the glycosaminoglycan in shake flask experiments

The transformed LMG-194 *E*. *coli araΔC* cells with pBAD plasmids harboring *Ph-CS-like* gene from *Pyrococcus* (LMG-pBAD-PhCS-like) were induced with 0.2% of L-arabinose. The LMG-194 *araΔC* with empty plasmid was served as a negative control for the production experiments. Each fresh transformant was grown overnight at 37°C with shaking at 235 rpm in SOB medium supplemented with 100 µg/mL of ampicillin and 10 µg/mL of doxycycline. Cultures were then diluted by 100 and grown to reach an optical density (OD₆₀₀) of 0.6 upon which gene expression was induced using 0.2% L-arabinose. The cultures were left to grow under 30°C for an additional 18 to 24 h.

### 1.1.2. Production of the glycosaminoglycan polymer in bioreactor

To increase the yield of GAG production, a fed-batch fermentation process was developed using a 2 L bioreactor (MINIFOR laboratory fermentor) in TB medium. In this fermentor, the culture conditions were controlled in term of temperature (37°C), pH (7), oxygenation (22) and agitation (4). Similarly, the transformed LMG-194 E. *coli* cells LMG-pBAD-PhCS-like were induced with 0.2% L-arabinose when reached an OD₆₀₀ of 0.6. Another strategy was used to increase the production was by adding 10 g.L⁻¹ glucose and 2.5 g.L⁻¹ phosphate in the fermentation culture of LMG-pBAD-PhCS-like after 6 h of induction with 0.2% L-arabinose.

### 1.2. GAG production in BL21 codon plus and C43 (DE3) recombinant strains

The transformed *E. coli* BL21 codon plus and C43 (DE3) with either empty pET102 vector or with the plasmids harboring *Ph-CS-like* gene from *Pyrococcus* were grown overnight at 37°C with shaking at 235 rpm in SOB medium supplemented with ampicillin (100 µg/mL) and chloramphenicol (25 µg/mL). The cultures were then diluted by 100 and grown to reach an optical density (OD₆₀₀) of 0.6 upon which gene expression was induced using 1 mM isopropyl-1-thio-β-D-galactoside (IPTG) purchased from Himedia. The cultures were left to grow under 30°C for an additional 18 to 24 h.

### 2. Downstream purification of the produced glycosaminoglycan

Fermentation brothes from cultures obtained after 24 h of induction were added to an equal volume of 0.1% of SDS and incubated at room temperature for 10 min and then centrifuged for 10 min at 6000 rpm. The obtained supernatant was treated with 2 volumes of cold absolute ethanol and incubated overnight at 4°C. The precipitate was then collected by centrifugation (3000 rpm for 10 min) and resuspended in 1/10 volume of 0.1 M NaCl by agitation for 10 min and the concentration of the obtained biopolymer was estimated by CTM assay based on the formation of turbidity between the negatively charged formed polyanionic polysaccharide and the cationic CTAB. Therefore, the turbidity resulting from the precipitation is titrated by spectrophotometry and is related to the concentration of the produced GAG.

The produced biopolymer was then subjected to different purification protocols to remove all the contaminants from the fermentation broth.
(1) Trichloroacetic acid and charcoal treatment: after proceeding with SDS treatment and ethanol precipitation, the nucleic acids and bacterial derived proteins were removed by lowering the pH of the broth from 6 to 2 by addition of trichloroacetic acid (10%) and subsequent charcoal treatment (2%) for 1 h followed by centrifugation at 7000 rpm for 30 min at 4°C. Then the supernatant was passed through a 0.45 µm filter and desalted in 300 kDa cut-off cassette (vivaspin 20 mL) then concentrated by adding 2 volume of ethanol to be further lyophilized for NMR analysis.
(2) Mechanical cell lysis: after 24 h of induction, the total fermentation broth was centrifuged for 10 min at 6000 rpm. The pellet was resuspended in 10% of 0.05 M NaCl and bacterial cells were lysed mechanically using a Microfluidics M110P apparatus with interaction chamber G10Z 67 µm at a working pressure of 20000 psi. The cells were then centrifuged for 1 h at 14000x g. The resulting supernatant was then treated with DNAse (1 mg/L), incubated for 1 h at 37°C.
(3) DEAE purification : It was shown that the purification of GAGs from crude cell is most commonly achieved by ion exchange chromatography after protease and nuclease digestion. After proceeding with SDS treatment and ethanol precipitation, samples were first treated with 1 mg/L of Pierce Universal Nuclease (Thermo SCIENTIFIC, Cat. No. 88700) and incubated for 1 h at 37°C for cell lysis and then with 2.5 mg/mL of proteinase K (Biotechrabbit, Cat. No. BR1100901) and incubated for 2 h at 56°C. Then each sample was passed through HiTrap Capto DEAE 5 mL with liquid chromatography AKTA^{™} system. The liquid chromatography system such as AKTA^{™} can separate substances based on their charges using an ion exchange resin containing positive charged group such as HiTrap Capto DEAE 5 mL. Fractionation of 10 mL was carried on by gradient elution from 10 to 50% of 1 M NaCl. Subsequently, these fractions were lyophilized to be further analyzed by different biochemical and spectroscopic methods.

### 3. Characterization of the produced glycosaminoglycan bipolymer

### 3.1. Biochemical characterization of the produced glycosaminoglycan

### 3.1.1. Identification of the produced glycosaminoglycan on Agarose gel

### 3.1.1.1. Separation of fractions on agarose gel

4. After purification by anion exchange chromatography (see "Downstream purification of the produced glycosaminoglycan » above), the resulting fractions were lyophilized to be further analyzed by agarose gel electrophoresis. 5 mg of each sample was added to 200 µL of loading dye (LD) containing bromophenol blue and formamide, then 10 µL was loaded in each well on 1% agarose gel. In addition, around 5 µg of standard HA or/and CS were loaded on the gel as standards for migration pattern and size. Following electrophoresis, the gels were stained with either stains-all for 24 h at room temperature to be then destained with 10% ethanol for 1 h or with 0.1% toluidine blue prepared in a solution of 1% acetic acid, 50% ethanol and 49% water and incubated for 15 min at room temperature to be then destained with the same solution without toluidine blue.

### 3.1.1.2. Analysis of the produced glycosaminoglycan by

### hyaluronidase digestion

The digestion of glycosaminoglycan was performed using hyaluronidase from bovine testes (Sigma-Aldrich cat. No. H 3506). This enzyme catalyzes the hydrolysis of (1-4) linkages between *N*-acetylhexosamine and D-glucuronate residues in chondroitin, chondroitin-4-sulfate, chondroitin-6-sulfate, and hyaluronic acid.

After purification by anion exchange chromatography, around 20 mg of the fraction containing the polymer of interest was dissolved in 100 µL of deionized water. 50 µL of each sample was treated with hyaluronidase (4 mg/mL) and both samples (with and without enzyme) were incubated for 24 h at 37°C. After lyophilization, 50 µL of deionized water was added to each sample and 20 µL was mixed with 10 µL of LD where 5 µL was loaded on the gel for analysis. The hyaluronidase was prepared at 1 mg/mL in HSE buffer (20 mM sodium phosphate buffer, pH 7.0, with 77 mM sodium chloride and 0.1 mg/mL BSA) or in 50 mM of ammonium acetate buffer (1.84 mL glacial acetic acid, 1.48 g sodium acetate in 350 mL H₂O, pH 4.5).

### 3.2. Structural characterization of the produced GAG

### 3.2.1. Analysis of the produced glycosaminoglycan by FTIR spectroscopy

After purification by anion exchange chromatography (see "Downstream purification of the produced glycosaminoglycan » above), the lyophilized fractions containing the polymer of interest eluted between 0.35 and 0.45 M of NaCl were applied to infrared radiation (IR) (Thermo-Nicolet, Avatar 320-FT-IR) to identify the functional groups and organic compounds of the produced GAG that could be attributed to the amid group (CONH), the sulfate group of D-galactosamine and the sulfonyl group (If the produced GAG was sulfated), and the carboxylate group. Around 10 mg of sample was typically sandwiched between two windows, separated by a known pathlength. Potassium bromide (KBr, spectroscopic grade) is typically used as the window material because it is transparent in the IR. Alternatively, samples can be contained within a KBr matrix and pressed to form a pellet that is then analysed. The signal processing is a Fourier transform. The acquisition conditions were as follow: Scanning from 0 to 4000 cm-1, 32 scans, resolution of 1 cm-1. Collection of the spectrum in Transmittance after acquisition of a spectrum background. For processing: simple FT then subtraction of the background spectrum from the product spectrum. Auto indexing of peaks or manual.

### 3.2.2. Analysis of GAG structure by NMR

Following the purification, the biopolymer produced from the two recombinant strains was subjected to physical analysis by one-dimensional 1H nuclear magnetic resonance (NMR). All NMR experiments were recorded on a Bruker Avance III 400 spectrometer operating at 400.13 MHz for ¹H, equipped with a BBFO probe with a Z-gradient coil and a GREAT 1/10 gradient unit. The standard temperature was adjusted to 298 K. Each experiment took around 15 min within 32 scan numbers. Around 10 mg of each sample was re-dissolved in 0.45 mL D₂O and transferred into a clean NMR microtubes. The data were processed by TopSpin (Bruker BioSpin) or MestReNova software. Hyaluronic acid sodium salt from *Streptococcus equi* (Sigma-Aldrich) and chondroitin sulfate C sodium salt (Carbosynth) were used as an NMR standards as bacterial glycosaminoglycan polysaccharides.

### 3.2.3. Mass spectrometry for the detection of disaccharides after hyaluronidase digestion

Mass spectrometry (MS) is a proven analytical method used to have information about the chemical structure of a chemical sample. Partial depolymerization of GAG chains using enzymes is an effective means of producing oligosaccharides that are amenable to MS analysis. MS analysis was performed on the chondroitin sulfate as well as the lyophilized fractions containing the polymer of interest before and after digestion with hyaluronidase (See paragraph 3.1.1.2). Following SDS treatment and purification by anion exchange chromatography, the lyophilized fractions containing the polymer of interest were diluted in a total volume of 100 µL in water and applied to MS, Shimadzu LCMS-2020 Prominence UFLC system with a single quadrupole mass spectrometer using ESI ionization (negative ion mode).

### RESULTS

### 1. Genetic engineering of E. coli strains to produce the glycosaminoglycan

### 1.1. Construction of E. coli Ph-CS-like recombinant strains in pBAD system

The pBAD expression system is used for the expression of heterologous genes in *E. coli* under the control of a weak promoter induced by L-arabinose. This system was chosen for the expression of the *PhCS-like* protein and production of its corresponding glycosaminoglycan because it is generally used for the production of biomolecules that results from a strong competition between cell growth and biomolecule accumulation. For instance, the pBAD system was successfully used for the production of recombinant hyaluronic acid by an *E. coli* strain expressing a HAS synthase from a *Streptococcus* ([1]). Recombinant *E. coli* clones possessing the *Ph-*z*CS-like* gene from *Pyrococcus horikoshii* were obtained by pBAD TOPO cloning (Invitrogen). These clones were designed to express the *PhCS-like* recombinant protein without the presence of additional Tags at the *N-*terminal and C-terminal that could alter the expression or activity of the heterologous enzyme once it is expressed in *E. coli* LMG-194 strain.

### 1.2. Cloning in pET system

The pET system offers an advantage over the pBAD system that it features a strong T7 promoter that permits high-level, IPTG inducible expression by which a recombinant protein can accumulate to up to 50% of total cellular proteins. This can be used to achieve a maximal yield of GAG production. The choice of vector and expression host can significantly increase the activity and the amount of target protein present in the soluble fraction. The pET102/D-TOPO^{®} vector generally allows to clone the gene of interest as a fusion to *N*-terminal His-Patch thioredoxin for increased translation efficiency and solubility of heterologous proteins. The thioredoxin is a 11.7 kDa protein that was originally isolated from *E. coli* as a hydrogen donor for ribonuclease reductase. When this protein is overexpressed in *E. coli,* it is able to accumulate to approximately 40% of the total cellular protein and still remain soluble. And interestingly, when used as a fusion partner, thioredoxin can increase translation efficiency. In the case of pET102/D-TOPO^{®}, the thioredoxin protein has been mutated to contain a metal binding domain, and is termed "His-Patch thioredoxin".

In addition, this system takes advantage of the high activity and specificity of the bacteriophage T7 RNA polymerase that allows regulated expression of heterologous genes in *E. coli* lambda DE3 lysogen which carries the gene for T7 RNA polymerase. Accordingly, two strains were used for production experiments, the BL21 codon plus and the C43 (DE3). The BL21 codon plus is generally used for high-level protein expression and easy induction in T7 expression systems. It harbors a prophage DE3 derived from a bacteriophage λ, which carries the T7 RNA polymerase gene under the control of the **lac**UV5 promoter. The cells harbor extra copies of tRNA coding genes (AUA, AGG, AGA, CUA, CCC and GGA) compensating for the rare tRNA and is used to overcome the expression problems coming from codon bias and therefore improves the expression of foreign protein. Such strain lacks also both the Lon protease and the OmpT protease that can degrade the expressed foreign protein. Whereas, the C43 (DE3) is usually effective for the expression of toxic and membrane proteins as it carries mutations that lower T7 RNAP expression levels that results in strongly reduced T7 RNAP accumulation levels. As a consequence, membrane protein production stress is alleviated, thereby increasing membrane protein yields without toxic effects.

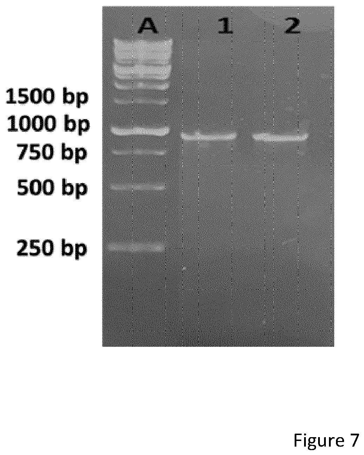

The DNA fragment coding for *Ph-CS-like* genes were successfully amplified by PCR, using the forward primers containing 4 base pair sequences (CACC) on the 5' end generating blunt-end PCR products that allow directional cloning, in a way such that our gene of interest will be optimally expressed and fused in frame with the vector for high-level, T7-regulated expression in *E. coli* (Figure 7). Following PCR amplification, both genes were cloned into pET vector and fused to *N*-terminal His-Patch thioredoxin for increased translation efficiency and solubility of heterologous proteins when expressed either in BL21 codon plus or in C43 (DE3). All the clones were selected on LB agar plates supplemented with ampicillin for selection and chloramphenicol to which BL21 codon plus and C43 (DE3) are resistant.

### 2. Expression of recombinant PhCS-like protein from Pyrococcus horikoshii

In order to detect the recombinant *PhCS-like* proteins expressed from the recombinant *E. coli* strains with molecular weight of 37 kDa (without Tag), culture in shake flask experiments were carried out. Overnight precultures of the transformed LMG-194 *E. coli araΔC* cells with the plasmids pBAD and BL21 codon plus and C43 (DE3) with the plasmids pET102 harboring *Ph-CS-like* gene were performed in SOB medium at 37°C. The cultures were then diluted 100 times with the same fresh medium supplemented with ampicillin for the selection of transformed bacteria and either doxycyline or chloramphenicol to wich LMG-194 or (DE3) strains are resistant, respectively. The cells were then incubated at 37°C to reach an optical density (OD) of 0.6 at 600 nm, upon which a final concentration of 0.2% of L-arabinose or 1 mM IPTG was added to induce the expression of the genes of interest in pBAD or pET expression system, respectively. Samples from non-induced and induced strains were taken at different times of induction, denatured at 95°C and loaded on the polyacrylamide gel for further separation on the basis of molecular masses. The Coomassie-brilliant blue was used to stain the proteins.

### 3. Production and characterization of the biopolymer

### 3.1. Bacterial GAG production in pBAD system

### 3.1.1. Production of the glycosaminoglycan polymer in shake flask experiments

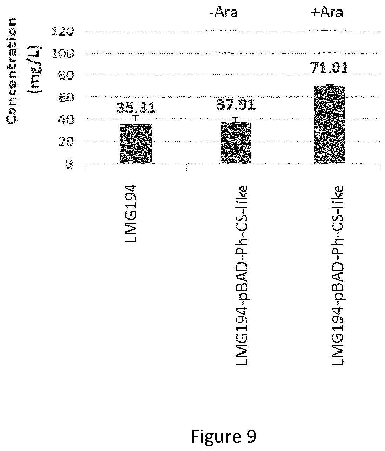

In order to evaluate the yield of the produced glycosaminoglycan in recombinant LMG-194 *E. coli* cells, culture in shake flask experiments were carried out. Overnight precultures of the transformed LMG-194 *E. coli araΔC* cells with the plasmid pBAD harboring *Ph-CS-like* gene (LMG-pBAD-PhCS-like) were performed in SOB medium at 37°C. The LMG-194 with empty plasmid served as a negative control for the production experiments. The cultures were then diluted 100 times with the same fresh medium supplemented with ampicillin for the selection of transformed bacteria and doxycyline to wich LMG-194 are resistant. The cells were then incubated at 37°C to reach an optical density (OD) of 0.6 at 600 nm, upon which a final concentration of 0.2% of L-arabinose was added to induce the expression of the genes of interest. The concentration of 0.2% L-arabinose was chosen based on previous experiments performed in the laboratory where it has been found that raising the concentration of L-arabinose above 0.2% did not result in further increase in the concentration of the obtained biopolymer as determined by the CTAB turbidimetry assay (CTM assay). This test is based on the formation of turbidity between the negatively charged polyanionic polymers (like HA and chondroitin) and CTAB, a cation surface active agent. The turbidity resulting from the precipitation is related to the polymer concentration and can be titrated by spectrophotometry and the concentration was calculated based on a HA standard curve. The temperature was then down shifted from 37 to 30°C after induction to allow better expression of the recombinant proteins. After 24 h of induction, the fementation broth was treated with SDS followed by ethanol precipitation and resuspension in NaCl to further determine the concentration of the produced GAG by the CTM assay. Induced and non-induced cultures were included to confirm that the produced polysaccharide is specific only to the induced-system. As expected, results showed that the concentration of the produced GAG was higher in the presence of L-arabinose, as it increased from 37.9 mg/L to 71.01 mg/L in LMG-pBAD-PhCS-like.. The strain with empty plasmid showed a concentration of 35.31 mg/L which is also very low compared to the induced strains (see Figure 9).

### 3.1.2. Production of the glycosaminoglycan polymer in a minifor 2L bioreactor

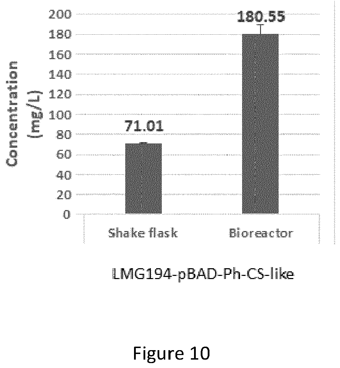

In order to increase the yield of production, GAG synthesis was carried out in a fed-batch fermentation process using 2 L bioreactor (MINIFOR laboratory fermentor) under defined conditions (temperature: 37°C, pH = 7, oxygenation: 22 and agitation: 4). The process transfers from shake flasks **to a** bioreactor had a visible effect on the concentration showing around 1 to 2-fold increase in GAG production, from 71.01 mg/L to 180.55 mg/L in LMG-pBAD-PhCS-like (see Figure 10).

### 3.2. Bacterial GAG production in pET system

### 3.2.1. GAG production by expression of PhCS-like protein in BL21 codon plus (DE3)

In order to estimate the yield of the produced GAG in the recombinant *E. coli* cells BL21 codon plus, culture in shake flask experiments were carried out. The transformed BL21 codon plus with either the empty pET102 vector (BL21 codonplus-pET102) or with the plasmids harboring *Ph-CS-like* gene (pET102-PhCS-like) were grown overnight at 37°C in SOB medium supplemented with ampicillin for the selection of bacteria transformed with pET102 plasmid containing the ampicillin resistance gene, and chloramphenicol to which BL21 (DE3) codon plus are resistant. The cultures were then diluted by 100 and grown to reach an optical density (OD) of 0.6 upon which gene expression was induced using 1 mM IPTG. The cultures were then left to grow under 30°C for an additional 18 to 24 h. Similarly, after SDS treatment and ethanol precipitation, the concentration of the produced GAG from fermentation broth was then estimated by CTM assay in the presence and absence of IPTG inducer. As above-mentioned, this test is based on the formation of turbidity between the negatively charged biopolymer and a CTAB, a cation surface active agent. The amount of turbidity is proportional to the amount of the produced GAG and the concentration was calculated based on a HA standard curve.

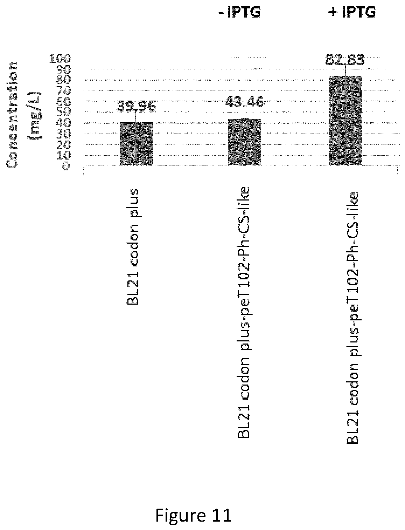

Results showed that GAG concentration was higher in the presence of IPTG, as it increased from 43.46 mg/L to 82.83 mg/L in pET102-PhCS-like. The concentration of polyanionic polymer produced by the negative control BL21 codonplus-pET1 02 harboring empty pET102 vector was 39.96 mg/mL, which revealed the presence of leakage in the system (Figure 11).

### 3.3. Characterization of the produced glycosaminoglycan

### 3.3.1. Purification of the produced bacterial GAG

In order to characterize the nature of the produced glycosaminoglycan in recombinant *E. coli* strains carrying *Ph-CS-like* genes from *P. horikoshii* bacterial cell cultures were subjected to different purification steps after 24 h of induction. This included SDS treatment followed by ethanol precipitation and protease and nuclease digestion. Then fractionation was carried out by anionic exchange chromatography using DEAE column that separates substances based on their charges by gradient elution with NaCl (See paragraph "Downstream purification of the produced glycosaminoglyca » in materials and methods). The resulting fractions were then lyophilized and analyzed by different biochemical and spectroscopic methods.

### 3.3.2. Biochemical characterization

### 3.3.2.1. Agarose gel electrophoresis

The agarose gel electrophoresis constitutes a sensitive method to visualize the non radiolabelled GAGs in submicrogram quantities in a complex polysaccharide mixture by sequential stains-all/toluidine blue staining. The carbocyanine based dye Stains-all (1-Ethyl-2-[3-(1-ethylnaphto[1,2-d]thiazoline-2-ylidene)-2-methyl-propenyl]naphto[1,2-d]thiazolium bromide) has been increasingly used to stain and identify the non-sulfated hyaluronic acid and other GAGs in submicrogram quantities with basis on their specific colors, at least on a screening level. In the case of GAGs, the non-sulfated HA was stained in bright-blue and the sulfated GAGs such as dermatan sulfate was stained in purple; heparin in yellow and keratan sulfate in light-red.

Following fractionation, the presence of the biopolymer in the resulting fractions was revealed on agarose gel electrophoresis by subsequent staining with the cationic carbocyanine dye Stains-All. The same conditions were applied to the wild type *E. coli* strain that served as negative control (N.C.) for the production experiments.

The analysis of fractions resulting from *E. coli* LMG-194 after DEAE purification showed that the produced GAG was present only in the induced recombinant strains and not in the wild type LMG-194 *E. coli* strain and the polymer was mainly concentrated in the fractions 14 and 15 eluted between 0.35 and 0.45 M of NaCl. Inspection of the migration pattern of the obtained GAG showed a faster migration pattern than a sample of purified commercial hyaluronic acid migrated along the samples. In addition, the color of the produced biopolymer corresponded more to the color code known for the chondroitin and not for hyaluronic acid. Both of the migration pattern and the color of the biopolymer revealed that it is more biochemically similar in composition to chondroitin than to hyaluronic acid.

Sequential staining with toluidine blue was also performed. This cationic dye is commonly used to detect GAGs due to its metachromatic property. It ususally stains sulfated polysaccharides in purple/pink in a specific manner and do not stain at all the non-sulfated polysaccharides. The negatively charged sulfates in the GAGs neutralize the positive charge of toluidine blue, leading to dye aggregation by hydrophobic bonding and van der Waals interactions.

Both GAGs standards as well as the fractions containing the polymer of interest from both recombinant *E. coli* strains were analyzed on agarose gel and stained with toluidine blue after electrophoresis. The results revealed the presence of hues of purple or dark blue in the standard commercial chondroitin sulfate sample as well as the purified fractions containing the polymer of interest.

The standard commercial HA was not stained. These results indicate the presence of a sulfated chondroitin produced from the recombinant strains expressing *Ph-CS-like* gene.

Similarly, the GAG polysaccharide resulting from the recombinant BL21 codon plus *E. coli* strain was analyzed after SDS treatement, ethanol precipitation and fractionation by DEAE purification. Results showed a contaminating polymer present in the induced and the negative control strain BL21 codon plus that could not be even resolved after purification by anion exchange chromatography. This could explain the presence of an endogenous polymer synthesized by *E.coli* BL21 codon plus itself and the production was not specific to the induced recombinant strain.

Therefore, LMG-194 strain using the inducible plasmid backbone of pBAD was the best choice to produce the following biopolymer. Further characterization methods were examined to identify the structure of the following GAG.

### 3.3.2.1.1. Analysis of the produced glycosaminoglycan by hyaluronidase digestion

The agarose gel electrophoresis can analyze all the complex GAGs and the specificities of the glycosaminoglycan-degrading enzymes. To go deeper into the nature of the produced GAG from the recombinant LMG-194 *E. coli* strain, enzymatic digestion by hyaluronidase (HAase) was performed on the purified fractions containing the polysaccharide of interest and analyzed by agarose gel electrophoresis after staining with Stains-all. In general, the hyaluronidase enzyme can hydrolyze randomly the (1-4)-linkages between *N*-acetyl-*β*-D-glucosamine and D-glucuronate residues in hyaluronate and the (1-4)-β-D-glycosidic linkages between *N*-acetyl-galactosamine or *N-*acetylgalactosamine sulfate and glucuronic acid in chondroitin, chondroitin 4- and 6-sulfates, and dermatan. In our case, the ability of HAase to attack the produced GAG by the recombinant *E*. *coli* strains would confer essential features about the nature of this polysaccharide.

Following SDS treatment, ethanol precipitation and purification by anion exchange chromatography (See paragraph "2. Downstream purification of the produced glycosaminoglycan" in materials and methods), the fractions containing the polysaccharide were treated with hyaluronidase and incubated for 24 h at 37°C. After lyophilization, samples were loaded on agarose gel for analysis. Interestingly, results showed that the produced GAG was clearly digested after 24 h of incubation with HAase. The chondroitin sulfate standard as well as the produced GAG took more time to be digested by hyaluronidase (24 h) when compared to the standard HA (1 h). Indeed, HAase is able to digest chondroitin sulfate at a slower rate than HA. This apparent retardation of digestion could confirm that the resulting biopolymer may have GlucNAcβ-(1-4)-Glucuronate, or GalNAc (sulfated or not) β-(1-4)-Glucuronate linkages.

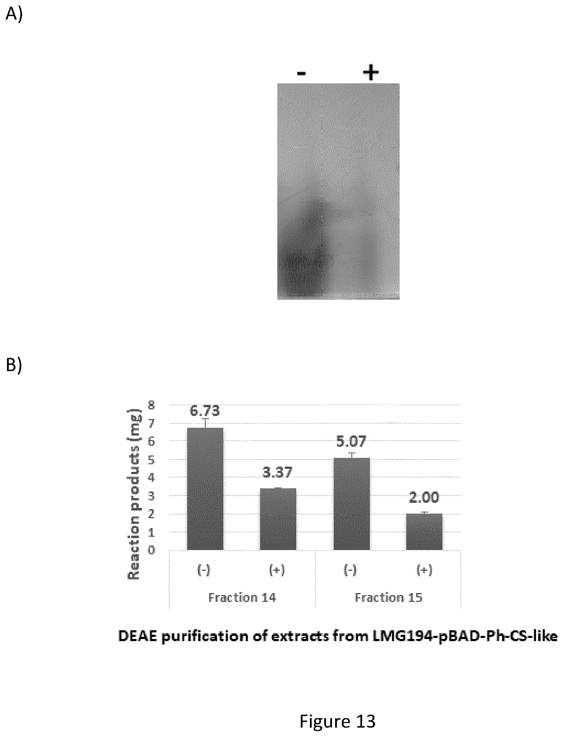

In parallel, CTM turbidity assay was performed on the lyophilized fractions 14 and 15, eluted between 0.35 and 0.45 M NaCl respectively by DEAE purification. This test was done to screen the hydrolysis activity of hyaluronidase against the produced GAG by which the amount of turbidity developed when CTAB is added is proportional to the amount of the formed reaction product. Similarly, the results were in agreement with those of agarose gel. As seen in Figure 13, the total mass of reaction product was decreased from 6.73 to 3.37 mg and from 5.07 to 2 mg in the fractions 14 and 15 respectively of LMG-pBAD-PhCS-like after 24 h of digestion with hyaluronidase.

In order to determine the chemical structure of the produced GAG from the recombinant LMG-194 *E. coli* strain, structural characterization including FTIR spectroscopy, NMR and mass spectrometry were performed on the lyophilized fractions containing the polymer resulting from anion exchange chromatography.

### 3.3.2.2. Analysis of the produced glycosaminoglycan by FTIR spectroscopy

In order to identify the functional groups and organic compounds of the produced GAG, FTIR spectroscopy was applied on the lyophilized fractions after DEAE purification containing the polysaccharide of interest produced from the recombinant *E. coli* strains LMG-pBAD-PhCS-like. This technique is based on measuring the infrared absorption of a test sample over a narrow range between 400-4000 cm⁻¹.

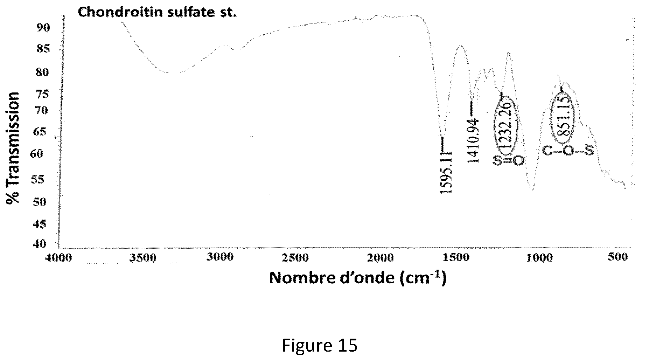

By comparing with the FTIR spectra of CS and standard bovine sodium chondroitin 4-sulphate reported by Sundaresan *et al.* (Sundaresan, G., R.J.J. Abraham, V. Appa Rao, R. Narendra Babu, V. Govind, and M.F. Meti, Established method of chondroitin sulphate extraction from buffalo (Bubalus bubalis) cartilages and its identification by FTIR. J Food Sci Technol, 2018. 55(9): p. 3439-3445 ([7])), a peak at 1646 cm⁻¹ that usually corresponds to -CONH was recorded in purified samples of LMG-pBAD-PhCS-like at 1648.36 cm⁻¹(Figure 15) (Table 11). Another peak that is usually found in 4-sulphate and 6-sulphate of D-galactosamine units attributed to C-O-S at 857 cm⁻¹ for standard CS was also recorded in samples of LMG-pBAD-PhCS-like at 863.78 cm⁻¹(Table 11) ([7]).

In addition, a peak that corresponds to S=O was observed at 1236 cm⁻¹ for standard CS ([7]), and same was recorded in the produced GAG from LMG-pBAD-PhCS-like at 1236.73 cm⁻¹. Another peak was recorded in extracted bacterial GAGs at 1419.96 cm⁻¹. These peaks correspond to C=O (-COO) vibration that was recorded at 1421 cm⁻¹ in the CS extracted from the bone of the tilapia spine (Table 8).

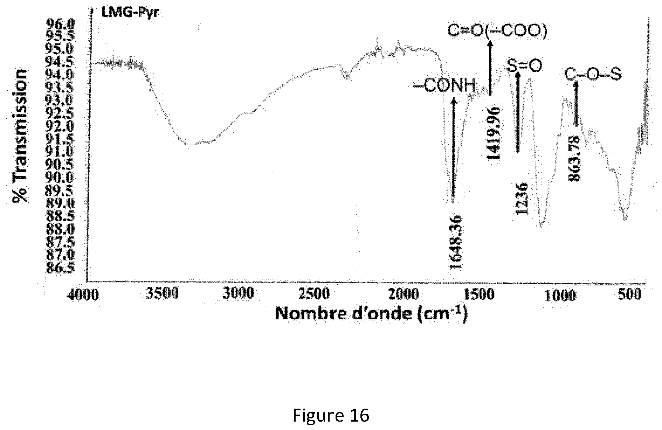

All these peaks were attributable also to the CS standard that was used in this study (Carbosynth), except for that at 1646 cm⁻¹ but this band remains between the range of 1739 1451 cm⁻¹ that represent the N-H (CH₃CO-NH-) structural vibration (Table 9) (Figure 16) (Oliveira, A., V. Feitosa, J. Oliveira, A. Coelho, L. Vieira, F.D.A. Rocha da Silva, F. Avelino, E. Duarte, B. Souza, and M. Filho, Characteristics of Chondroitin Sulfate Extracted of Tilapia ( Oreochromis niloticus) Processing. Procedia Engineering, 2017. 200: p. 193-199 ([8])). It is worth noting that the variations in the source and the physical condition of samples can produce significant differences in peak position. Moreover, interestingly the peaks corresponding to C-O-S and S=O at 860 and 1236 cm⁻¹ respectively were very intense compared to the reference chondroitin, confronting our hypothesis that the produced GAG is sulfated. In addition, by comparing these spectra with standard reference HA from *Streptococcus equi,* we found that there are no peaks close to the peaks at 860 and 1236 cm⁻¹, supporting our interpretation concerning the presence of sulfate (Table 11).

**Table 11: FTIR analysis showing the peak number and positions (in terms of wave number, cm-1). The peak number and positions were shown in CS and HA GAG standards, and the produced GAG from the recombinant E. coli strains LMG-pBAD-PhCS-like using FTIR spectroscopy.**

| | Chondroi tin sulfate (CS) (referred to literature ) | Chondroitin sulfate C sodium salt (Carbosynth ) | LMG-pBAD-PhCS-like | Contrib ution | HA standard from *Streptococ cus equi* | Contribution |
|---|---|---|---|---|---|---|
| Wave number (cm⁻¹) | 1236 | 1232.26 | 1236.73 | S=O | 1075.51 | C-O-C |
| | 857 | 851.15 | 863.78 | C-O-S | 1407.13 | C-O/C=O |
| | 1421 | 1410.94 | 1419.96 | C=O(-COO) | 1606.90 | Amid II group |
| | 1646 | 1595.11 | 1648.36 | - CONH | 2881.42 | C-H |
| | | | | | 3268.97 | OH |

### 3.3.2.3. Characterization of GAG structure by NMR analysis

In order to determine the chemical structure of the produced GAG, NMR analysis was performed first on the lyophilized total cell extracts resulting from the fermentation of recombinant *E. coli* strains carrying *Ph-CS-like* from *Pyrococcus* after SDS treatment and ethanol precipitation.

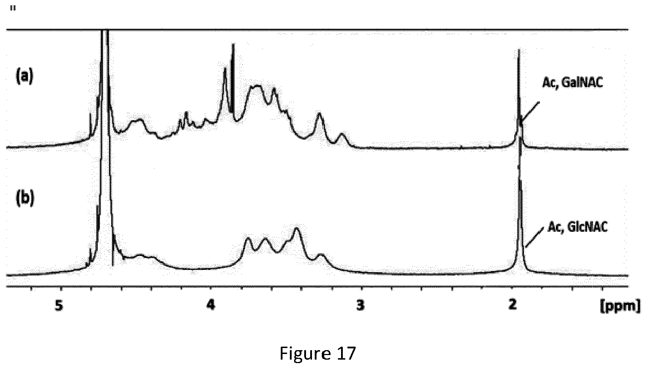

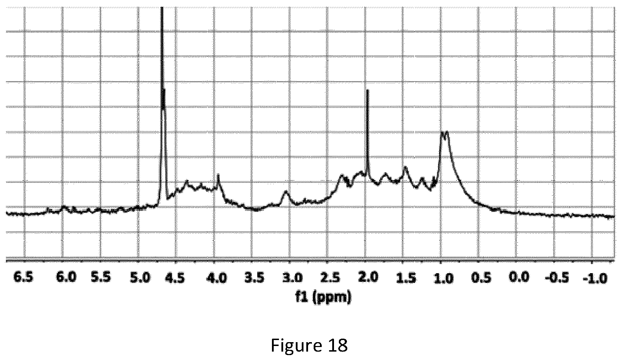

In comparaison to HA and chondroitin sulfate standards (Figure 17), an important shift in the positions of proton signals in the region of sugar between 3 and 4 ppm was noticed in polysaccharides purified from recombinant strains . Moreover, the signature peak attributed to the proton of acetyl group in *N*-acetylglucosamine in both standard GAGs at 1.9 ppm was not observed in both extracts. More distinct peaks also appeared with high intensity as the doublet of doublets at 2.56 ppm that can be attributed to the fatty acyl chains of phospholipids and between 0.7 and 2 ppm that can be attributable to alkyl groups of phospholipids (Figure 18). NMR of phosphate was then carried out and showed that phosphate was highly present in the extract mixture indicating the high level of phospholipids that altered the characterization of the produced GAG.

Accordingly, different isolation and downstream purification processes were employed to remove all the contaminants in crude samples for efficient recovery of GAG from the fermentation broth to maximize NMR sensitivity. These approaches included (1) trichloroacetic acid and charcoal treatment to remove nucleic acids and bacterial derived proteins (2) mechanical lysis of cells to prevent the use of SDS (3) SDS treatment and ethanol precipitation followed by anion exchange chromatography described above.

The best purification method was SDS treatment, followed by ethanol precipitation and anionic exchange column DEAE (See paragraph "2. Downstream purification of the produced glycosaminoglycan" in materials and methods). After lyophylization, NMR analysis was performed on the purified and lyophilized extracts from both recombinant E. coli strains. The results showed that both polymers exhibited the same characteristic peak at 1.9 ppm (Figure 18) attributed to the proton of acetyl group of either *N-*acetylglucosamine in HA or *N*-acetylgalactosamine in CS proving that this recombinant GAG is acetylated.

In addition, NMR analysis of the purified extracts was performed on the resulting digest products after hyaluronidase digestion. However, the proton signals corresponding to hyaluronidase were predominant since this enzyme is a glycoprotein that in turn masked the appearance of oligosaccharides even in CS standard. Therefore, we moved toward mass spectrometry for the detection of oligosaccharides resulting from hyaluronidase digestion.

### 3.3.2.4. Mass spectrometry for the detection of disaccharides after hyaluronidase digestion

In order to evaluate the GAG disaccharides resulting from hyaluronidase digestion of the purified glycosaminoglycan produced from both recombinant LMG-194 *E*. *coli* strain, we performed mass spectrometry (MS) by which we can determine the molecular weight of each isolated component. After purification by anion exchange chromatography and hyaluronidase digestion, the lyophilized samples as well as CS standard were applied to MS for analysis.

The eight biologically relevant chondroitin sulfate disaccharides are listed in the Table 12 below. The mass spectrum of each disaccharide generates one predominant ion (listed as m/z).

**Table 12:**

| | | | | |
|---|---|---|---|---|
| **Chondroitin Disaccharides** | | | | |
| 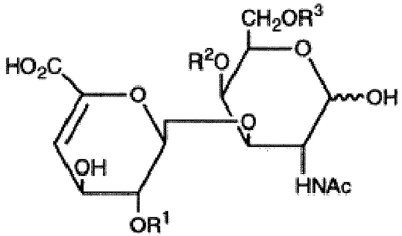 | | | | |

| disach. | R¹ | R² | R³ | m/z |
|---|---|---|---|---|
| UA-GalNAc | H | H | H | 378 |
| UA 2S-GalNAc | SO₃ | H | H | 458 |
| UA-GalNAc4S | H | SO₃ | H | 458 |
| UA-GalNAc6S | H | H | SO₃ | 458* |
| UA2S-QalNAc4S | SO₃ | SO₃ | H | 268.5 |
| UA2S-GalNAc6S | SO₃ | H | SO₃ | 268.5 |
| UA-GalNAc4S, 6S | H | SO₃ | SO₃ | 268.5* |
| UA2S-GalNAc4S, 6S | SO₃ | SO₃ | SO₃ | 205.3* |

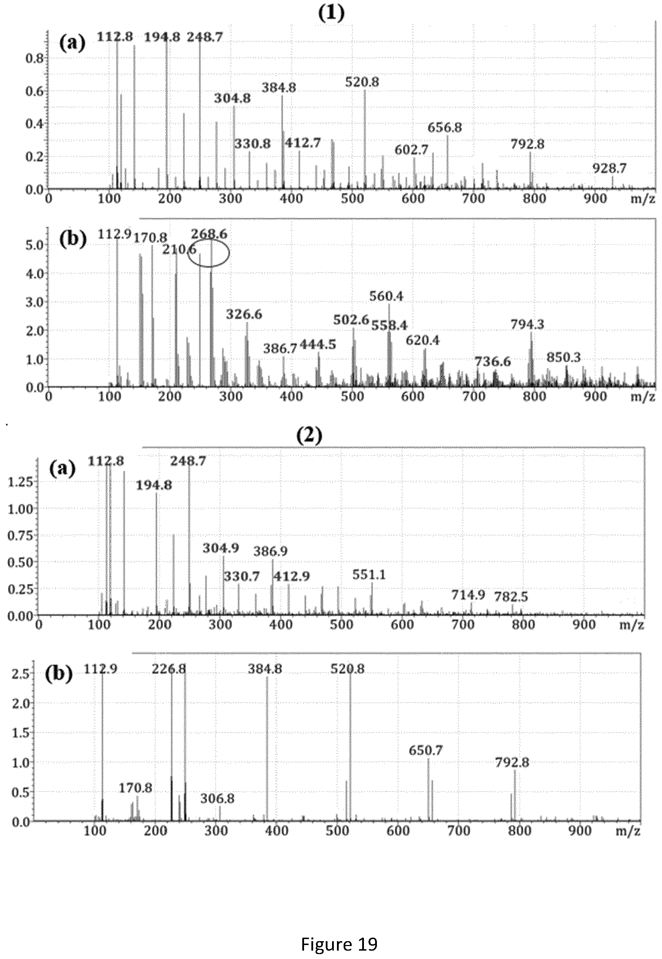

The MS analysis of CSC standard did not show significant changes in the background before and after digestion. The major molecular ions with *m*/*z* values of 248.7 ; 466.8 ; 112.8 ; 194.8 ; 412.8 ; 602.7 ; 330.8 ; 118.9 were predominant before and even after hyaluronidase digestion (Figure 19 20). This could be due to the endolytic activity of HAase that in turn resulted in longer polysaccharides that could not be detected in our MS conditions. However, these predominant peaks were not found in both samples that share peaks with *m*/*z* values at 268.6 ; 326.6 ; 444.5 ; 502.6 ; 558.5; 736 .3 that either completely disappeared after hyaluronidase digestion or had a lower intensity (Table 12). Interestingly, both samples share the peak with *m*/*z* value of 268.6 that corresponded to chondroitin sulfate disaccharide (Table 10). This peak disappeared completely in LMG-pBAD-PhCS-like.Moreover, we can clearly notice significant changes in the spectrum profile of both samples during digestion, meaning that a polymer with a structure related to this class of GAG is present.

### Reference List

1. Yu, H. and G. Stephanopoulos, Metabolic engineering of Escherichia coli for biosynthesis of hyaluronic acid. Metab Eng, 2008. 10(1): p. 24-32.
2. He W, Fu L, Li G, Andrew Jones J, Linhardt RJ, Koffas M. Production of chondroitin in metabolically engineered E. coli. Metab Eng. 2015;27:92-100
3. Kumar, S. and R. Nussinov, How do thermophilic proteins deal with heat? Cellular and Molecular Life Sciences CMLS, 2001. 58(9): p. 1216-1233.
4. Camacho, C., G. Coulouris, V. Avagyan, N. Ma, J. Papadopoulos, K. Bealer, and T.L. Madden, BLAST+: architecture and applications. BMC Bioinformatics, 2009. 10: p. 421.
5. Remmert, M., A. Biegert, A. Hauser, and J. Söding, HHblits: lightning-fast iterative protein sequence searching by HMM-HMM alignment. Nat Methods, 2011. 9(2): p. 173-5.
6. Guex, N., M.C. Peitsch, and T. Schwede, Automated comparative protein structure modeling with SWISS-MODEL and Swiss-PdbViewer: a historical perspective. Electrophoresis, 2009. 30 Suppl 1: p. S162-73.
7. Sundaresan, G., R.J.J. Abraham, V. Appa Rao, R. Narendra Babu, V. Govind, and M.F. Meti, Established method of chondroitin sulphate extraction from buffalo (Bubalus bubalis) cartilages and its identification by FTIR. J Food Sci Technol, 2018. 55(9): p. 3439-3445.
8. Oliveira, A., V. Feitosa, J. Oliveira, A. Coelho, L. Vieira, F.D.A. Rocha da Silva, F. Avelino, E. Duarte, B. Souza, and M. Filho, Characteristics of Chondroitin Sulfate Extracted of Tilapia (Oreochromis niloticus) Processing. Procedia Engineering, 2017. 200: p. 193-199.
9. Kubaski, F., H. Osago, R.W. Mason, S. Yamaguchi, H. Kobayashi, M. Tsuchiya, T. Orii, and S. Tomatsu, Glycosaminoglycans detection methods: Applications of mass spectrometry. Mol Genet Metab, 2017. 120(1-2): p. 67-77.
10. Liu, Z., F. Zhang, L. Li, G. Li, W. He, and R.J. Linhardt, Compositional analysis and structural elucidation of glycosaminoglycans in chicken eggs. Glycoconj J, 2014. 31(8): p. 593-602.
11. Bi, Y., C. Hubbard, P. Purushotham, and J. Zimmer, Insights into the structure and function of membrane-integrated processive glycosyltransferases. Curr Opin Struct Biol, 2015. 34: p. 78-86.
12. Sambrook et al , MOLECULAR CLONING: A LABORATORY MANUAL, 2nd ed., Cold Spring Harbor, 1989, and 3rd ed., 2001.
13.Studier, F.W.: "Protein production by auto-induction in high density shaking cultures", Protein Expr. Purif., 2005, 41, 207-34.2005.

## Claims

1. Isolated gene coding for the enzyme Glycosyl transferase 2 from *Pyrococcus horikoshii* OT3, the nucleotide sequence of said gene comprising or consisting of sequence SEQ ID NO : 1, or its homologs from hyperthermophilic *Archaea.*

2. Isolated enzyme Glycosyl transferases 2 encoded by the isolated gene according to claim 1.

3. Isolated gene according to claim 1, or isolated *enzyme* according to claim 2, wherein said homologs are selected from *Thermococcus sibiricus, Thermococcus kodakarensis, Thermococcus onnurineus, Pyrococcus abyssi, Pyrococcus furiosus, Pyrococcus sp. (strain NA2), Thermococcus gammatolerans, Pyrococcus yayanosii, Halorhabdus tiamatea, Natrialba magadii, Halorhabdus utahensis, Methanosarcina acetivorans, Methanosarcina barkeri, Halorubrum lacusprofundi, Natrialba magadii, Halogeometricum borinquense, Thermococcus gammatolerans and Thermococcus barophilus.*

4. Plasmid vector comprising a gene coding for the enzyme Glycosyl transferase 2 from *Pyrococcus horikoshii* OT3, the nucleotide sequence of said gene comprising or consisting of the sequence SEQ ID NO : 1, or its homologs from hyperthermophilic *Archaea.*

5. Plasmid vector according to claim 4, also comprising elements allowing the expression of said gene in a cell.

6. Plasmid vector according to claim 4 or 5, comprising a promoter selected from an arabinose inducible promoter and a T7 inducible promoter, operationally linked to said isolated gene.

7. Plasmid vector according to claim 6, wherein said arabinose inducible promoter is a weak inducible promoter, and said T7 inducible promoter is a strong inducible promoter.

8. Plasmid vector according to claim 7, also comprising a sequence coding for the enzyme UDP-glucose dehydrogenase KFOF, preferably in tandem with said isolated gene.

9. Plasmid vector according to claim 8, also comprising a sequence coding for the enzyme UTP-glucose-1-P-Uridyltransferase *GALU.*

10. Plasmid vector according to claim 5, comprising or consisting of nucleotide sequence SEQ ID NO: 3 or SEQ ID NO : 4.

11. A host cell comprising a recombinant polynucleotide encoding Glycosyl transferase 2 from *Pyrococcus horikoshii* OT3, or its homologs from hyperthermophilic Archaea.

12. A host cell according to claim 11, said host cell being *E. Coli.*

13. A host cell according to claim 12, wherein *E. Coli* is selected among ΔaraC E.coli cells, in particular LMG194 *E. Coli* strain, BL21 (DE3), BL21 (DE3) codon plus, BL21 (C41) and BL21 (C43) *E. Coli* strains.

14. A host cell according to anyone of claims 11 to 13, wherein the recombinant polynucleotide comprises sequence SEQ ID NO : 1 or a plasmid vector as defined in anyone of claims 4 to 10.

15. Process for producing sulfated glycosaminoglycans (GAGs), said method comprising the steps of:
a) culturing a host cell as defined in anyone of claims 11 to 14 in a culture medium containing glucose or any sugar or sugar acid synthesized from glucose, and under conditions compatible with the production of said GAGs by said cell; and
b) recovering sulfated GAGs from the culture medium of step (a).

16. Sulfated glycosaminoglycans obtained by a process as defined in claim 15.

17. Use of a sulphated GAG as defined in claim 16, for producing pharmaceutical, cosmetic, nutraceutical or food products.
